# EUROPEAN PATENT SPECIFICATION

(11) **EP 1 603 914 B1**
(45) Date of publication and mention of the grant of the patent: **17.01.2007**
(21) Application number: 04713892.0
(22) Date of filing: 24.02.2004
(51) Int. Cl.: C07D 487/04, C07D 495/04, A61K 31/407, A61P 25/00, C07D 209/00, C07D 333/00

(54) **COMPOUNDS HAVING ACTIVITY AT 5HT2C RECEPTOR AND USES THEREOF**
VERBINDUNGEN MIT EINER 5-HT2C REZEPTOR-WIRKUNG UND IHRE VERWENDUNG
COMPOSES AYANT UNE ACTIVITE AU NIVEAU DU RECEPTEUR 5HT2C ET LEURS UTILISATIONS

(30) Priority: 11.03.2003 GB 0305553
(43) Date of publication of application: 14.12.2005
(73) Proprietor: GLAXO GROUP LIMITED, Greenford, Middlesex UB6 ONN (GB)
(72) Inventor: HAMPRECHT, Dieter, GlaxoSmithKline, I-37135 Verona (IT); MICHELI, Fabrizio, GlaxoSmithKline, I-37135 Verona (IT)
(74) Representative: Dolton, Peter Irving Ernest
(86) International application number: PCT/EP2004/001844
(87) International publication number: WO 2004/081010

(56) References cited:
- WO-A-94/04533
- WO-A-97/48700
- WO-A-03/089409

## Description

This invention relates to novel compounds having pharmacological activity, processes for their preparation, to compositions containing them and to their use in the treatment and/or prevention of CNS disorders and other disorders.

WO 96/23783, WO 97/48699 and WO 97/48700 all disclose a series of indoline derivatives which are 5-HT_{2C} receptor antagonists and which are claimed to be useful in the treatment of various CNS disorders.

A novel class of compounds possessing 5-HT_{2C} receptor activity has been found. The present invention therefore provides, in a first aspect, a compound of formula (I) or a pharmaceutically acceptable salt thereof: 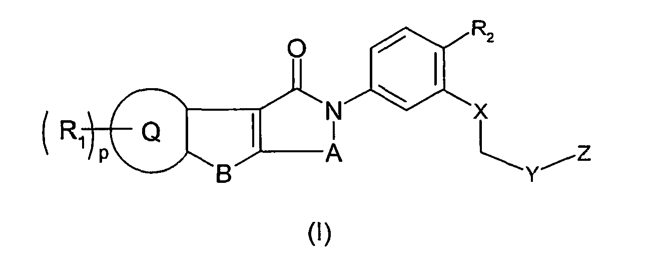 wherein:
Q is phenyl or a 6-membered heteroaromatic group containing at least one nitrogen atom;
A is -(CH₂-CH₂)-, -(CH=CH)-, -(CH₂)₃-, -C(CH₃)₂-, -(CH=CH-CH₂)-, or a group -(CHR₃)-wherein R₃ is hydrogen, halogen, hydroxy, cyano, nitro, C₁₋₆alkyl, C₃₋₇cycloalkyl, C₃₋₇ cycloalkyloxy, haloC₁₋₆alkyl, C₁₋₆alkoxy, haloC₁₋₆alkoxy or C₁₋₆alkylthio;
B is O, S or NR₁₁, wherein R₁₁ is hydrogen, C₁₋₆alkyl optionally substituted by C₁₋₆alkoxy, or is C₁₋₆alkanoyl optionally substituted by C₁₋₆alkoxy;
R₁ is halogen, cyano, C₁₋₆alkyl, C₃₋₇cycloalkyl, C₃₋₇cycloalkyloxy, C₁₋₆alkoxy, C₁₋₆alkylthio, hydroxy, amino, mono- or di-C₁₋₆alkylamino, an N-linked 4 to 7 membered heterocyclic group, nitro, haloC₁₋₆alkyl, haloC₁₋₆alkoxy, aryl, arylC₁₋₆alkyl, arylC₁₋₆alkyloxy, arylC₁₋₆alkylthio, COR₄ (wherein R₄ is amino, mono- or di-C₁₋₆alkylamino or an N-linked 4 to 7 membered heterocyclic group), COOR₅ or COR₆ (wherein R₅ and R₆ are independently hydrogen or C₁₋₆alkyl);
p is 0, 1 or 2 or 3;
R₂ is hydrogen, halogen, hydroxy, cyano, nitro, C₁₋₆alkyl, C₁₋₆alkanoyl, C₃₋₇cycloalkyl, C₃₋₇cycloalkyloxy, haloC₁₋₆alkyl, C₁₋₆alkoxy, haloC₁₋₆alkoxy, C₁₋₆alkylthio, amino, mono- or di-C₁₋₆alkylamino or an N-linked 4 to 7 membered heterocyclic group;
X is oxygen, sulfur, -CH₂- or NR₈ wherein R₈ is hydrogen or C₁₋₆alkyl;
Y is a single bond, -CH₂-, -(CH₂)₂- or -CH=CH-; and
Z is an optionally substituted N-linked heterocyclic group or a C-linked 4 to 7 membered heterocyclic group containing at least one nitrogen, or Z is -NR₉R₁₀ where R₉ and R₁₀ are independently hydrogen or C₁₋₆alkyl.

The following terms, whether used alone or as part of another group are to be given the following meanings, unless otherwise stated.

The term "6-membered heteroaromatic group containing at least one nitrogen atom" refers to groups such as pyridyl, pyridazinyl, pyrinidinyl, pyrazinyl and triazinyl.

The term "halogen" and its abbreviated form "halo" are used herein to describe fluorine, chlorine, bromine or iodine.

The term "alkyl" is used herein to describe a straight chain or branched fully saturated hydrocarbon group. "C₁₋₆alkyl" refers to alkyl groups having from one to six carbon atoms, including all isomeric forms, such as methyl, ethyl, propyl, isopropyl, butyl, isobutyl, sec-butyl, tert-butyl, pentyl, neopentyl, sec-pentyl, n-pentyl, isopentyl, tert-pentyl and hexyl.

The term "C₁₋₆alkanoyl" refers to an alkanoyl group having from 1 to 6 carbon atoms, such as methanoyl (or "formyl"), ethanoyl (or "acetyl"), propanoyl, isopropanoyl, butanoyl, isobutanoyl, sec-butanoyl, pentanoyl, neopentanoyl, sec-pentanoyl, isopentanoyl, tertpentanoyl and hexanoyl.

The term "C₁₋₆alkoxy" refers to a straight chain or branched chain alkoxy (or "alkyloxy") group having from one to six carbon atoms, including all isomeric forms, such as methoxy, ethoxy, propoxy, isopropoxy, butoxy, isobutoxy, sec-butoxy, tert-butoxy, pentoxy, neopentoxy, sec-pentoxy, n-pentoxy, isopentoxy, tert-pentoxy and hexoxy.

The term "C₃₋₇cycloalkyl" refers to a cycloalkyl group consisting of from 3 to 7 carbon atoms, such as cyclopropane, cyclobutane, cyclopentane, cyclohexane and cycloheptane. Optional substituents for C₃₋₇cycloalkyl includes one or more halogen, hydroxy, oxo, C₁₋₆alkyl, cyano, CF₃, OCF₃, C₁₋₆alkoxy and C₁₋₆alkanoyl.

The term "C₁₋₆alkylthio" refers to a straight chain or branched chain alkylthio group having from one to six carbon atoms, such as methylthio, ethylthio, propylthio, isopropylthio, butylthio, isobutylthio, sec-butylthio, tert-butylthio, pentylthio, neopentylthio, sec-pentylthio, n-pentylthio, isopentylthio, tert-pentylthio and hexylthio.

The term "mono- or di-C₁₋₆alkylamino" refers to an amino group which is substituted by one C₁₋₆alkyl group or an amino group which is substituted by two C₁₋₆alkyl groups, the two amino groups being the same or different. Examples of monoC1-6alkylamino include methylamine, ethylamine, propylamine, isopropylamine, butylamine, isobutylamine, sec-butylamine, tert-butylamine, pentylamine, neopentylamine, sec-pentylamine, n-pentylamine, isopentylamine, tert-pentylamine and hexylamine. Examples of di-C1-6alkylamino include dimethylamine, diethylamine, dipropylamine, diisopropylamine, dibutylamine, diisobutylamine, disec-butylamine, ditert-butylamine, dipentylamine, dineopentylamine, dihexylamine, butylmethylamino, isopropylmethylamino, ethylisopropylamino, ethylmethylamino, etc.

The term "aryl" is used herein to describe 5 to 7-membered monocyclic or 9 to 11-membered bicyclic aromatic carbocyclic groups such as phenyl or naphthyl, or monocyclic or bicyclic heteroaromatic groups such as pyrrolyl, furyl, thienyl, pyrrolinyl, imidazolyl, pyrazolyl, pyrazolinyl, thiazolyl, isoxazolyl, furazanyl, pyridyl, pyridazinyl, pyrimidinyl, pyrazinyl, indolyl, isoindolyl, indazolyl, indolinyl, isoindolinyl, benzimidazolyl, benzoxazolyl, benzothienyl, quinolyl, quinoxalinyl, quinazolinyl and isoquinolyl; all of which may be optionally substituted by one or more of C₁₋₆alkyl (to form "arylC₁₋₆alkyl"), halogen, CF₃ or C₁₋₆alkoxy (to form "arylC₁₋₆alkoxy").

The terms "halo C₁₋₆alkoxy" or "haloC₁₋₆alkyl" are used to describe a C₁₋₆alkoxy or a C₁₋₆alkyl group, respectively, substituted with one or more halogens. Examples include - CHCl₂, -CF₃, -OCF₃, etc.

The term "heterocyclic group" is used herein to describe an aromatic or non-aromatic ring containing 1, 2 or 3 heteroatoms selected from nitrogen, sulphur and oxygen. Suitable examples of 4 to 7 membered heterocyclic groups include azetidinyl, pyrrolidinyl, imidazolidinyl, pyrazolidinyl, oxazolinyl, isothiazolidinyl, thiazolidinyl, pyrrolyl, pyrrolinyl, pyrazolinyl, imidazolyl, pyrazolyl, isothiazolyl, thiazolyl, piperidyl, piperazinyl, morpholinyl, pyridyl, pyridazinyl, pyrimidinyl, pyrazinyl, azepinyl, azepanyl, dioxolanyl, thienyl, tetrahydrothienyl, tetrahydrofuryl, dioxanyl and dithianyl.

The term "N-linked heterocyclic group" is used herein to describe a heterocyclic group which is linked to the remainder of the molecule via a nitrogen atom. Suitable examples of 4 to 7 membered N-linked heterocyclic groups include azetidinyl, pyrrolidinyl, imidazolidinyl, pyrazolidinyl, oxazolinyl, isothiazolidinyl, thiazolidinyl, pyrrolyl, pyrrolinyl, pyrazolinyl, imidazolyl, pyrazolyl, piperidyl, piperazinyl, morpholinyl and azepanyl.

The term "C-linked heterocyclic group containing at least one nitrogen" is used herein to describe a heterocyclic group which is contains at least one nitrogen atom and is linked to the remainder of the molecule via a carbon atom. Suitable examples of 4 to 7 membered C-linked heterocyclic groups containing at least one nitrogen include azetidinyl, pyrrolidinyl, imidazolidinyl, pyrazolidinyl, pyrrolyl, pyrrolinyl, pyrazolinyl, imidazolyl, pyrazolyl, piperidyl, piperazinyl, morpholinyl, pyridyl, pyridazinyl, pyrimidinyl, pyrazinyl, azepinyl and azepanyl.

More than one optional substituent may be present in the N-linked or C-linked heterocycle, which may be the same or different, and may be attached to any carbon atom of the heterocycle or an available nitrogen atom.

Suitable optional substituents for the N-linked or C-linked heterocycle include C₁₋₆alkyl, amino, mono- or di- C₁₋₆alkylamino, aryl, arylC₁₋₆alkyl, arylamino, hydroxy, C₁₋₆alkylamido, hydroxyC₁₋₆alkyl, C₁₋₆alkoxycarbonyl, halogen, haloC1-6alkyl, a heteroaromatic group (such as indole or benzimidazole), an aromatic or non-aromatic N-linked or C-linked heterocycle or an aromatic or non-aromatic heterocycleC₁₋₆alkyl optionally substituted by C₁₋₆alkyl. Examples of aromatic or non-aromatic heterocycleC₁₋₆ alkyl include heterocycle-methyl (such as pyridinyl-methyl and benzimidazolyl-methyl) and heterocycle-ethyl (such as morpholinyl-ethyl and indolyl-ethyl).

Substituents in the N-linked or C-linked heterocycle may form a bridge structure, to form a group such as for example 2-oxa-5-azabicyclo[2.2.1]heptyl. Such a bicyclic group may be further substituted by the substituents listed above. More than one substituent may be present on the same carbon atom to form spiro structures such as 1,4 and 1,5 dioxa spiro compounds.

When A is a group -(CHR₃)-, preferably R₃ is hydrogen. Preferably A is -CH₂-.

When p is 2 or 3, R₁ may be the same or different. Preferably p is 1 or 2 and R₁ is/are halogen, particularly chloro or fluoro.

Preferably R₂ is C₁₋₆alkoxy (particularly methoxy), halogen or cyano.

Preferably X is oxygen.

Preferably Y is -CH₂-.

Preferably Z is an optionally substituted N-linked 4 to 7 membered heterocycle, in particular piperidyl. Preferred substituents include halogen (particularly fluoro) and C₁₋₆ alkyl (particularly methyl).

Preferred compounds are compounds of formula (Ia): 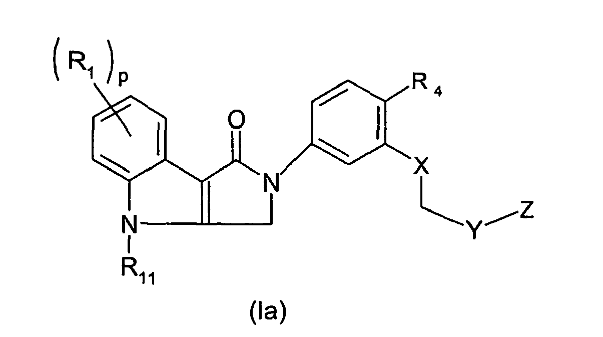 wherein R₁, p, R₁₁, R₄, X, Y and Z, are as defined for formula (I). Preferred features of formula (I) also apply to formula (Ia).

Preferred compounds include:
6-Bromo-5-methoxy-2-[4-methoxy-3-(2-piperidin-1-yl-ethoxy)phenyl]-8-methyl-1,8-dihydro-2H-2,8-diazacyclopenta[a]inden-3-one hydrochloride
5-Methoxy-2-[4-methoxy-3-(2-piperidin-1-yl-ethoxy)phenyl]-8-methyl-1,8-dihydro-2H-2,8-diazacyclopenta[a]inden-3-one hydrochloride
5,6-Dichloro-2-[4-methoxy-3-(2-piperidin-1-yl-ethoxy)phenyl]-8-methyl-1,8-dihydro-2H-2,8-diazacyclopenta[a]inden-3-one hydrochloride
5-Chloro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
2-[4-Methoxy-3-(2-piperidin-1-yl-ethoxy)phenyl]-1,2-dihydro-8-thia-2-azacyclopenta[a]inden-3-one hydrochloride
2-{4-Methoxy-3-[2-(4-methyl-piperidin-1-yl)-ethoxy]-phenyl}-1,2-dihydro-8-thia-2-azacyclopenta[a]inden-3-one hydrochloride
2-[4-Methoxy-3-(2-piperidin-1-yl-ethoxy)phenyl]-8-methyl-1,8-dihydro-2H-2,8-diazacyclopenta[a]inden-3-one hydrochloride
5-Chloro-2-[4-methoxy-3-(2-piperidin-1-yl-ethoxy)phenyl]-8-methyl-1,8-dihydro-2H-2,8-diazacyclopenta[a]inden-3-one hydrochloride
6-Chloro-2-[4-methoxy-3-(2-piperidin-1-yl-ethoxy)phenyl]-8-methyl-1,8-dihydro-2H-2,8-diazacyclopenta[a]inden-3-one hydrochloride
6-Fluoro-2-[4-methoxy-3-(2-piperidin-1-yl-ethoxy)phenyl]-8-methyl-1,8-dihydro-2H-2,8-diazacyclopenta[a]inden-3-one hydrochloride
2-{4-Methoxy-3-[2-(4-methyl-piperidin-1-yl)-ethoxy]-phenyl}-1,8-dimethyl-1,8-dihydro-2H-2,8-diazacyclopenta[a]inden-3-one hydrochloride
2-{4-Methoxy-3-[2-(4-methyl-piperidin-1-yl)-ethoxy]-phenyl}-8-methyl-1,8-dihydro-2H-2, 8-diazacyclopenta[a]inden-3-one hydrochloride
6-Chloro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
5-Fluoro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
6-Fluoro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
7-Fluoro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
and pharmaceutically acceptable salts thereof.

The compounds of formula (I) can form acid addition salts. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid.

The compounds of this invention may be in crystalline or non-crystalline form, and, if crystalline, may optionally be hydrated or solvated. This invention includes within its scope stoichiometric hydrates as well as compounds containing variable amounts of water.

Certain compounds of formula (I) are capable of existing in stereoisomeric forms (e.g. geometric or *("cis-trans")* isomers, diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of formula (I) or a pharmaceutically acceptable salt thereof, which process comprises:
(a) reacting a compound of formula (II): 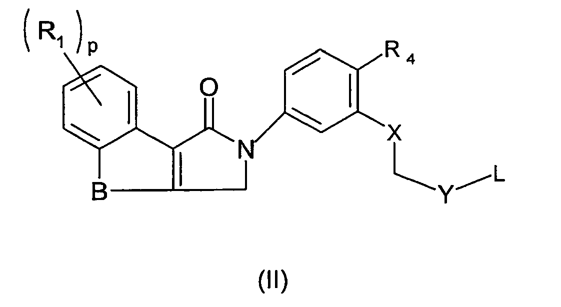 wherein R₁, R₂, p, A, X, and Y are as defined for formula (I), and L is a leaving group, with a compound of formula (III):

   Z-H (III)

   wherein Z is as defined for formula (I); or
(b) reacting a compound of formula (III): 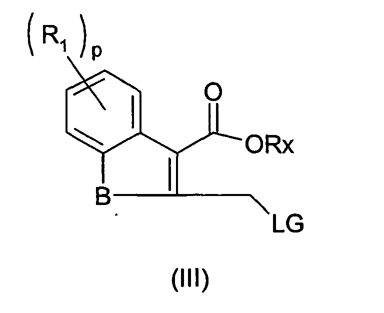 with a compound of formula (IV) or a salt thereof: 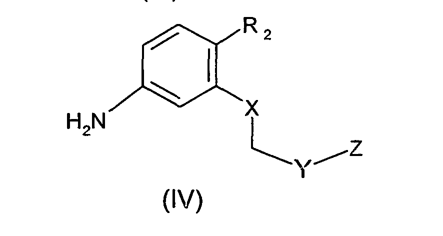 followed, as appropriate, with a treatment of the intermediate obtained with AlMe₃ or a similar oxophilic reagent;
   and for either process (a) or process (b), optionally followed by:
   - removing any protecting groups; and/or
   - converting a compound of formula (I) into another compound of formula (I); and/or
   - forming a pharmaceutically acceptable salt.

For the reaction of process (a), suitably L is mesylate. The reaction may take place in a solvent such as DMF in the presence of sodium iodide and potassium carbonate.

For the reaction of process (b), LG is a leaving group such as halogen, for example chloro or bromo, or a sulfonate ester, for example mesyl or tosyl. Rx is alkyl, for example Me. The reaction may take place in a solvent such as DMF at elevated temperature, suitably 100 °C. Where this reaction does not lead to spontaneous cyclisation of the intermediate alkylation product this material is treated with AlMe₃ or a similar oxophilic reagent.

Compounds of formula (I) can be converted into further compounds of formula (I) using standard techniques. For example, and by way of illustration rather than limitation, a compound wherein A is -(HCOH)- may be converted to a compound wherein A is -(CH₂)-by using a suitable reducing agent such as triethylsilane-trifluoroacetic acid using dichloromethane as solvent;

Compounds of formulae (II), (III) and (IV) are commercially available or may be prepared according to methods described herein or may be prepared according to known methods or by analogous methods thereto.

Those skilled in the art will appreciate that it may be necessary to protect certain groups to carry out the above processes. Suitable protecting groups and methods for their attachment and removal are conventional in the art of organic chemistry, such as those described in Greene T.W. 'Protective groups in organic synthesis' New York, Wiley (1981).

Pharmaceutically acceptable salts may be prepared conventionally by reaction with the appropriate acid or acid derivative.

In another aspect, the present invention provides a pharmaceutical composition comprising a compound of formula (I) or a pharmaceutically acceptable salt thereof, and a pharmaceutically acceptable carrier or excipient.

In a further aspect, the present invention provides a process for preparing a pharmaceutical composition, the process comprising mixing a compound of formula (I) or a pharmaceutically acceptable salt thereof and a pharmaceutically acceptable carrier or excipient.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents (e.g. pregelatinised maize starch, polyvinylpyrrolidone or hydroxypropyl methylcellulose);, fillers (e.g. lactose, microcrystalline cellulose or calcium hydrogen phosphate);, tabletting lubricants lubricants (e.g. magnesium stearate, talc or silica);, disintegrants (e.g. potato starch or sodium starch glycollate); and acceptable wetting agents (e.g. sodium lauryl sulphate). The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents (e.g. sorbitol syrup, cellulose derivatives or hydrogenated edible fats), emulsifying agents (e.g. lecithin or acacia), non-aqueous vehicles (which may include edible oils e.g. almond oil, oily esters, ethyl alcohol or fractionated vegetable oils), preservatives (e.g. methyl or propyl-p-hydroxybenzoates or sorbic acid), and, if desired, conventional flavourings or colorants, buffer salts and sweetening agents as appropriate. Preparations for oral administration may be suitably formulated to give controlled release of the active compound.

For parenteral administration, fluid unit dosage forms are prepared utilising a compound of the invention or pharmaceutically acceptable salt thereof and a sterile vehicle. Formulations for injection may be presented in unit dosage form e.g. in ampoules or in multi-dose, utilising a compound of the invention or pharmaceutically acceptable salt thereof and a sterile vehicle, optionally with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device. Thus compounds of formula (I) may be formulated for oral, buccal, parenteral, topical (including ophthalmic and nasal), depot or rectal administration or in a form suitable for administration by inhalation or insufflation (either through the mouth or nose).

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

The compounds of the present invention have affinity for the 5-HT_{2C} receptor. The affinity can be determined by assessing their ability to displace [³H]-mesulergine from rat or human 5-HT_{2C} clones expressed in 293 cells *in vitro,* as described in WO 94/04533.

All the Example compounds were tested according to this assay and were found to have pKi values >5.8. Some compounds show a considerably higher affinity in the range of 7.0 to >9.0 in human cells.

The intrinsic activity of the compounds of this invention can be determined according to the [³⁵S]GTPγS functional assay which is described in WO 99/07700.

Compounds of formula (I) and their pharmaceutically acceptable salts are of use in the treatment of certain CNS disorders such as depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type, vascular dementia with depressed mood, schizoaffective disorder or the depressed type, and depressive disorders resulting from general medical conditions including, but not limited to, myocardial infarction, diabetes, miscarriage or abortion, *etc*), anxiety including generalised anxiety and social anxiety disorder, schizophrenia, panic disorder, agoraphobia, social phobia, epilepsy, obsessive compulsive disorder and post-traumatic stress disorder, pain (particularly neuropathic pain), migraine, memory disorders, including dementia, amnesic disorders and age-associated memory impairment, disorders of eating behaviours including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. cannabis, heroin, morphine), sedative ipnotic, amphetamine or amphetamine-related drugs (e.g. dextroamphetamine, methylamphetamine) or a combination thereof, Alzheimer's disease, motor disorders such as Parkinson's disease, dementia in Parkinson's disease, neuroleptic-induced Parkinsonism and tardive dyskinesias, as well as other psychiatric disorders, disorders associated with spinal trauma and/or head injury such as hydrocephalus, gastrointestinal disorders such as IBS (Irritable Bowel Syndrome), Crohn's disease, ulcerative colitis, non-steroidal antiinflammatory drug induced damage) as well as microvascular diseases such as macular oedema and retinopathy.

It is to be understood that, as used herein, the term "treatment" refers to alleviation of established symptoms as well as prophylaxis.

Thus the present invention also provides a compound of formula (I) or a pharmaceutically acceptable salt thereof, for use as a therapeutic substance. In particular, the present invention provides a compound of formula (I) or a pharmaceutically acceptable salt thereof for use in the treatment of the above disorders. In particular the invention provides a compound of formula (I) or a pharmaceutically acceptable salt thereof for use as a therapeutic substance in the treatment of a CNS disorder. Preferably the CNS disorder is depression and/or anxiety.

Compounds of the invention may be administered in combination with other active substances such as 5HT3 antagonists, NK-1 antagonists, serotonin agonists, selective serotonin reuptake inhibitors (SSRI), noradrenaline re-uptake inhibitors (SNRI), tricyclic antidepressants and/or dopaminergic antidepressants.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRls which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

It will be appreciated that the compounds of the combination or composition may be administered simultaneously (either in the same or different pharmaceutical formulations), separately or sequentially.

The invention further provides a method of treatment of the above disorders in mammals including humans, which comprises administering to the sufferer a therapeutically safe and effective amount of a compound of formula (I) or a pharmaceutically acceptable salt thereof. In particular the invention provides a a method of treatment of a CNS disorder in mammals including humans, which comprises administering to the sufferer a therapeutically safe and effective amount of a compound of formula (I) or a pharmaceutically acceptable salt thereof. Preferably the disorder is depression and/or anxiety.

In another aspect, the invention provides for the use of a compound of formula (I) or a pharmaceutically acceptable salt thereof in the manufacture of a medicament for use in the treatment of the above disorders. In particular the present invention provides the use of a compound of formula (I) or a pharmaceutically acceptable salt thereof in the manufacture of a medicament for use in the treatment of a CNS disorder. Preferably the CNS disorder is depression and/or anxiety.

The composition of the present invention may contain from 0.1 % to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three times a day. Such therapy may extend for a number of weeks or months. When administered in accordance with the invention, no unacceptable toxicological effects are expected with the compounds of the invention.

All publications, including but not limited to patents and patent applications, cited in this specification are herein incorporated by reference as if each individual publication were specifically and individually indicated to be incorporated by reference herein as though fully set forth.

The following Descriptions and Examples illustrate the preparation of compounds of the present invention.

### General Procedure 1 (G1): Hydrochloride salt formation

To a solution of the free base in CH₂Cl₂ was added excess HCl (1 M in Et₂O). The resulting mixture was evaporated to dryness and the residue triturated using a mixture of EtOAc:Et₂O *ca.* 1:1 and dried to give the respective hydrochloride salt.

### General Procedure 2 (G2): Bromination to form substituted bromomethyl heterobicyclic compounds

A mixture of the substituted methyl heterocyclic compound, *N*-bromosuccinimide (typically 1.1 eq.) and benzoyl peroxide (0.05 eq.) in dry CCl₄ (2 ml per mmol substituted toluene) was heated at reflux. After complete conversion (typically 5 h; NMR control) the mixture was allowed to cool, filtered (washing with CH₂Cl₂ :petroleum ether *ca*. 1:1), concentrated and submitted to column chromatography to give the respective substituted bromomethyl heterocyclic compound.

### General Procedure 3 (G3): Methyl ester formation

To the substituted heterobicyclic carboxylic acid in dry CH₂Cl₂ *(ca.* 0.2-0.5 M) containing catalytic DMF *(ca.* 0.1 eq.) at 0 °C was added with stirring oxalyl chloride *(ca.* 1.3 eq.). The ice bath was removed. After gas evolution had ceased (typically 1-2 h) volatiles were removed *in vacuo.* To the residue was added dry methanol (to give a *ca.* 0.2 - 1 M solution; CAUTION: on scales above *ca.* 5 mmol this involved a significant exotherme) and the mixture was kept for 16 h or alternatively heated at 60 °C for 2 h. The material obtained after evaporation of the volatiles was used as such or purified by column chromatography.

### General Procedure 4 (G4): N-Methylation

To the substituted indole in dry DMF *(ca.* 0.5 M) was added methyl iodide (1 eq.) followed by sodium hydride (60% in mineral oil, 1.2 eq.). CAUTION: on scales above *ca*. 2 mmol this involved a significant exotherme. After 3 h water was added with stirring and the product of acceptable purity for further conversion, as appropriate, either collected by filtration and washed subsequently with water, a few drops of MeOH, and petroleum ether or
obtained from the extraction of this mixture using petroleum ether:EtOAc:CH₂Cl₂ 2:1:1, evaporation of the volatiles, and trituration of the residue with petroleum ether.

### General Procedure 5 (G5): Chlorination to form substituted chloromethyl heterobicyclic compounds

A mixture of the substituted methyl heterocyclic compound was treated as described in Procedure G2 but using *N*-chlorosuccinimide instead of *N*-bromosuccinimide to give the respective substituted chloromethyl heterocyclic compound.

### General Procedure 6 (G6): Preparation of substituted 2-(2-nitro-phenyl)-3-hydroxy-but-2-enoic acid methyl ester compounds

Typical scale 5 mmol: To NaH (60% in mineral oil, 2.3 eq.) in dry DMF (20 ml per 1 g 60% NaH) at 0 °C was added portionwise over 20 min via syringe with vigorous stirring acetylacetate methyl ester (2.1 eq.). After 10 min this mixture was allowed to warm to 25 °C and added to a stirred reaction vessel cooled in an ice bath containing the substituted 2-nitro-fluorobenzene (1.0 eq.). After 45 min the mixture was allowed to warm to 25 °C. After 16 h excess aqueous HCl (2 M) was added followed by water and Et₂O. The layers were mixed, to give the substituted 2-(2-nitro-phenyl)-3-hydroxy-but-2-enoic acid methyl ester compounds.

### General Procedure 7 (G7): Preparation of substituted indoles using TiCl₃

To a stirred mixture of the substituted 2-(2-nitro-phenyl)-3-hydroxy-but-2-enoic acid methyl ester compound in HOAc (6.5 ml per mmol) was added a solution of TiCl₃ (15% in 20-30% aqueous HCl, 6.5 ml per mmol). The resulting mixture was heated to 90 °C for 1.5 h, then cooled in an ice bath. Water and CH₂Cl₂ :MeOH 9:1 were added with stirring. The organic layer was collected, diluted with EtOAc:petroleum ether 1:1, washed (brine *2) and concentrated. The desired substituted indoles were obtained in adequate purity for further conversion following either trituration (Et₂O:petroleum ether 1:5) or column chromatography.

### Preparation 1 (P1): 1-[2-(2-Methoxy-5-nitro-phenoxy)-ethyl]-piperidine

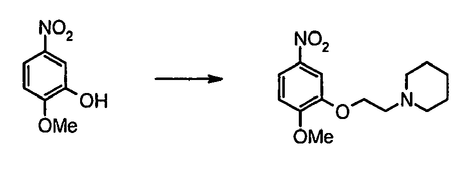

To a solution of 2-methoxy-5-nitrophenol (10 g) in DMF (45 ml) at room temperature were added K₂CO₃ (23 g) and 1-(2-chloroethyl)piperidin hydrochloride (12 g). The suspension was stirred at room temperature for 3 days. The suspension was diluted with water and twice extracted with EtOAc: Et₂O 1:2, the combined organic phases were washed with brine, concentrated to dryness *in vacuo*, to obtain the title product as a brown oil (99%). **NMR (¹H, CDCl₃):** δ 7.85 (dd, 1H), 7.75 (d, 1 H), 6.90 (d, 1 H), 4.20 (t, 2H), 3.90 (s, 3H), 2.80 (t, 2H), 2.45-2.60 (m, 4H), 1.55-1.65 (m, 4H) (m, 4H), 1.35-1.50 (m, 2H).
**MS (*m*/*z*):** 281 [MH]⁺.

### Preparation 2 (P2): 4-Methoxy-3-(2-piperidin-1-yl-ethoxy)-phenylamine (hydrochloride)

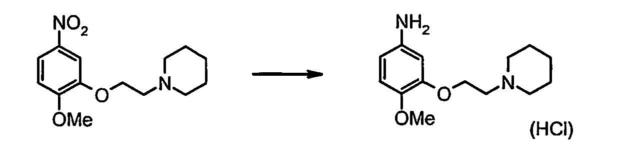

To 1-[2-(2-methoxy-5-nitro-phenoxy)-ethyl]-piperidine (59 mmol) in a mixture of MeOH (140 ml), NH₄Cl (17.1 g), water (140 ml) and conc. aqueous HCl (4.9 ml) was added iron powder (10.6 g). The mixture was heated at reflux with vigorous stirring for 90 min. and concentrated to *ca.* half volume *in vacuo.* EtOAc was added, filtered (Celite), washing with water. Et₂O was added, the layers were mixed and the aqueous layer collected. K₂CO₃ was added until pH *ca.* 9-10 and the mixture was twice extracted with EtOAc:Et₂O 1:1. The combined organic layers were dried (K₂CO₃), filtered and concentrated to give the free base of the title compound as a red oil (56 mmol). **NMR (¹H, CDCl₃):** δ 6.67 (d, 1H), 6.30 (d, 1H), 6.20 (dd, 1 H), 4.07 (t, 2H), 3.75 (s, 3H), 3.40 (bs, 2H), 2.80 (t, 2H), 2.40-2.55 (m, 4H), 1.51-1.62 (m, 4H), 1.35-1.46 (m, 2H).
Batches of this material were converted to the hydrochloride salt as required by treating a solution of the free base in dichloromethane with 1 eq. HCl (1 M solution in Et₂O), followed by evaporation to dryness.

### Preparation 3 (P3): 2-(2-Methoxy-5-nitro-phenoxy)-1-(4-methyl-piperidin-1-yl)-ethanone

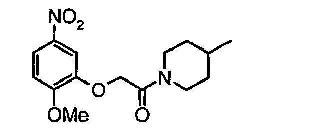

To a NaOH solution (14 g of NaOH, 350 mmol, in 300 ml of water) at 0 °C containing 4-metylpiperidine (36 ml, 310 mmol) chloroacetylchloride (23.7 ml, 305 mmol) was added in 10 min under vigorous stirring at 0 °C. After 3 h the mixture was acidified using HCl (2M aqueous) and the acqueous phase was extracted twice with CH₂Cl₂ (2 x 150 ml). The combined organic extracts were dried over anhydrous Na₂SO₄, filtered and concentrated to dryness *in vacuo* to give 37 g of intermediate which was dissolved in N-methyl pyrrolidone (120 ml).
To this solution 2-methoxy-5-nitrophenol (24 g, 156 mmol), K₂CO₃ (21 g, 152 mmol) and Nal (2 g, 13 mmol) were added. The suspension was mechanically stirred and heated at 120 °C. After 16 h the reaction was cooled and diluted with Et₂O (500 ml), the organic phase was washed twice with water (2x 150 ml), then with NaOH (aqueous, 1M, 100 ml) and finally with brine (150 ml).
The organic phase was dried over anhydrous Na₂SO₄, filtered and concentrated to dryness *in vacuo* to give 35 g of the title compound as a yellow solid. **NMR (¹H, CDCl₃):** δ 8.00 (dd, 1H), 7.76 (d, 1 H), 6.98 (d, 1 H), 4.87 (s, 2H), 4.55 (d, 1 H), 4.02 (s, 3H), 3.85 (d, 1 H), 3.13 (t, 1 H), 2.68 (t, 1 H), 1.55-1.85 (m, 4H), 1.10-1.30 (m, 1 H), 1.01 (d, 3H). **MS (*m*/*z*):** 309 [MH]⁺.

### Preparation 4 (P4): 1-[2-(2-Methoxy-5-nitro-phenoxy)-ethyl]-4-methyl-piperidine

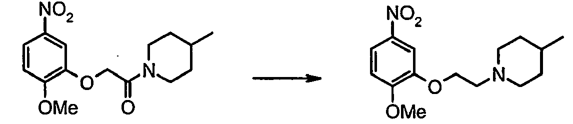

2-(2-Methoxy-5-nitro-phenoxy)-1-(4-methyl-piperidin-1-yl)-ethanone (11 g, 35.7 mmol) was dissolved in anh. THF (20 ml), 1M BH₃.THF (2.2eq, 78.5 ml) was added dropwise to the solution, and the mixture was heated at reflux for 4 hours. The solution was cooled to room temperature, CH₃OH (100 ml) was added, and the solvent was removed under reduced pressure. The residue was dissolved in CH₃OH (100 ml), 6N HCl (200 ml) was added, and the solution was heated to reflux for 30'. The CH₃OH was removed under reduced pressure and the remaining aqueous solution was made basic (pH>10) with 2.5 M NaOH. The basic solution was extracted with ethyl acetate (3x80 ml). The combined organic extracts were dried over anhydrous Na₂SO₄ and the solvent was removed under reduced pressure to give 9.4 g of the title product as a red oil. (yield: 90%). **NMR (¹H, CDCl₃):** δ 7.91 (dd, 1H)**,** 7.80 (d, 1 H), 6.90 (d, 1 H), 4.21 (t, 2H), 3.96 (s, 3H), 2.97 (m, 2H), 2.85 (t, 2H), 2.12 (m, 2H), 1.64 (m, 2H), 1.4 (m, 1 H), 1.29 (m, 2H), 0.92 (d, 3H). **MS (*m*/*z*)**: 295 [MH]⁺.

### Preparation 5 (P5): 4-Methoxy-3-[2-(4-methyl-piperidin-1-yl)-ethoxy]-phenylamine (hydrochloride)

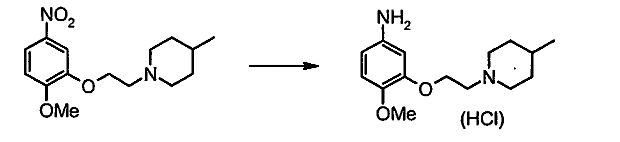

To a solution of 1-[2-(2-methoxy-5-nitro-phenoxy)-ethyl]-4-methyl-piperidine (9.5 g, 32.3 mmol) in abs. EtOH (200 ml) were added Pd/C 10% (1 g) and the reaction mixture was hydrogenated at room temperaturature at 1 atm. After 5 hrs the reaction mixture was filtered on a Celite pad and the solution was concentrated *in vacuo* to give 8.5 g of the title product as a brown solid (yield: 99%). **NMR (¹H, CDCl₃):** δ 6.61 (d, 1H), 6.26 (d, 1H), 6.04 (dd, 1H), 4.60 (bs, 2H), 3.91 (t, 2H), 3.58 (s, 3H), 2.85 (m, 2H), 2.61 (t, 2H), 1.97 (m, 2H), 1.52 (m, 2H), 1.3 (m, 1 H), 1.1 (m, 2H), 0.86 (d, 3H).
Batches of this material were converted to the hydrochloride salt as required as described for Preparation 2.

### Preparation 6 (P6): 5-Methoxy-1,2-dimethyl-1H-indole-3-carboxylic acid methyl ester

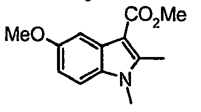

The title compound was obtained from 5-methoxy-1,2-dimethyl-1*H* indole-3-carboxylic acid according to Procedure G3 (quant. yield): **NMR (¹H, CDCl₃):** δ 7.60 (s, 1 H), 7.15 (d, 1H), 6.84 (d, 1 H), 3.88 (s, 3H), 3.85 (s, 3H), 3.64 (s, 3H), 2.72 (s, 3H). **m.p.:** 123-126 °C.

### Preparation 7 (P7): 6-Bromo-2-bromomethyl-5-methoxy-2-methyl-1H-indole-3-carboxylic acid methyl ester

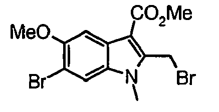

The title compound was obtained from 5-methoxy-1,2-dimethyl-1*H*-indole-3-carboxylic acid methyl ester according to Procedure G2 (using 1.2 eq. N-bromosuccinimide, 8% yield): **NMR (¹H, CDCl₃):** δ 7.65 (s, 1H), 7.55 (s, 1H), 5.09 (s, 2H), 3.94 (s, 6H), 3.73 (s, 3H).

### Preparation 8 (P8): 1,2-Dimethyl-1H-indole-3-carboxylic acid ethyl ester

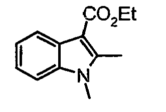

The title compound was obtained from 2-methyl-1*H*-indole-3-carboxylic acid ethyl ester according to Procedure G4 (yellow powder, 87% yield): **NMR (¹H, CDCl₃):** δ 8.08-8.12 (m, 1 H), 7.16-7.31 (m, 3H), 4.37 (q, 2H), 3.70 (s, 3H), 2.77 (s, 3H), 1.44 (t, 3H).

### Preparation 9 (P9): 2-Chloromethyl-1-methyl-1H-indole-3-carboxylic acid ethyl ester

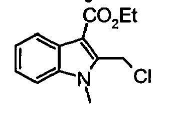

The title compound was obtained from 1,2-dimethyl-1*H*-indole-3-carboxylic acid ethyl ester (P8, 0.45 g) according to Procedure G5 (yellow oil that solidified on cooling, 94% yield): **NMR (¹H, CDCl₃):** δ 8.15 (d, 1 H), 7.23-7.36 (m, 3H), 5.28 (s, 2H), 4.40 (q, 2H), 3.83 (s, 3H), 1.44 (t, 3H).

### Preparation 10 (P10): 2-(4,5-Dichloro-2-nitro-phenyl)-3-hydroxy-but-2-enoic acid tert-butyl ester

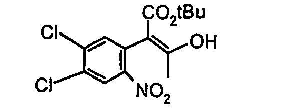

To acetylacetate *tert*-butyl ester (1.6 ml) in dry DMF (10 ml) at 0 °C was added portionwise with vigorous stirring NaH (60% in mineral oil, 0.46 g). After 15 min at 25 °C the mixture was again cooled in an ice bath and 1,2-dichloro-4-fluoro-5-nitrobenzene was added in a single portion resulting in a dark purple solution. This was allowed to warm in the defrosting ice bath over 2.5 h. Aqueous HCl (1 M, 7.5 ml) was added followed by water and Et₂O. The layers were mixed, the organic layer collected, washed (brine), concentrated and submitted to column chromatography to give the title compound as a yellow oil (1.7 g): **NMR (¹H, CDCl₃):** δ 13.2 (s, 1 H), 8.08 (s, 1 H), 7.35 (s, 1 H), 1.88 (s, 3H), 1.32 (s, 9H). **MS (*m*/*z*):** 346 [M-H]⁻ (2Cl).

### Preparation 11 (P11): 5,6-Dichloro-2-methyl-1H-indole-3-carboxylic acid tert-butyl ester

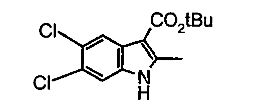

To 2-(4,5-dichloro-2-nitro-phenyl)-3-hydroxy-but-2-enoic acid *tert-*butyl ester (P10, 4.8 mmol) in a mixture of MeOH (12 ml), NH₄Cl (1.4 g), water (12 ml) and ethanol (10 ml) was added iron powder (0.86 g). The mixture was heated at reflux with vigorous stirring for 2.5 h and concentrated to *ca.* half volume *in vacuo.* EtOAc was added, filtered (Celite), washing with water and EtOAc. The organic layer was collected, volatiles evaporated *in vacuo* and the residue submitted to column chromatography to give, besides the corresponding *N*-hydroxy indole derivative (major product, 64%), the title compound as a yellow solid (0.19 g, 13%). **NMR (¹H, CDCl₃):** δ 8.27 (bs, 1H), 8.13 (s, 1H), 7.32 (s, 1H), 2.69 (s, 3H), 1.62 (s, 9H). **MS (*m*/*z*):** 298 [M-H]⁻ (2Cl).

### Preparation 12 (P12): 5,6-Dichloro-1,2-dimethyl-1H-indole-3-carboxylic acid tert-butyl ester

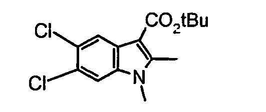

The title compound was obtained from 5,6-dichloro-2-methyl-1*H*-indole-3-carboxylic acid *tert-*butyl ester (P11, 0.19 g) according to Procedure G4 (colourless solid, 86% yield):
**NMR (¹H, CDCl₃):** δ 8.17 (s, 1H). 7.35 (s, 1 H), 3.64 (s, 3H), 2.71 (s, 3H), 1.62 (s, 9H).

### Preparation 13 (P13): 2-Chloromethyl-5,6-dichloro-1-methyl-1H-indole-3-carboxylic acid tert-butyl ester

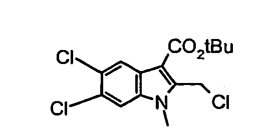

The title compound was obtained from 5,6-dichloro-1,2-dimethyl-1*H*-indole-3-carboxylic acid *tert*-butyl ester (P12, 0.17 g) according to Procedure G5 (yellow solid that solidified on cooling, 96%): **NMR (¹H, CDCl₃):** δ 8.23 (s, 1 H), 7.43 (s, 1 H), 5.21 (s, 2H), 3.77 (s, 3H), 1.64 (s, 9H).

### Preparation 14 (P14): 5-Chloro-2-methyl-1H-indole-3-carboxylic acid methyl ester

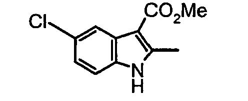

The title compound was obtained by:
1. Procedure G6 but using 2,4-dichloronitrobenzene (5 mmol) instead of a substituted 2-nitro-fluorobenzene. The reaction required heating (2 h at 60 °C followed by 5 h at 85 °C). The product was obtained as a yellow oil (0.88 g) containing impurities as judged by NMR.
2. Treating this material as described in Procedure G7 to give the title compound as an off-white solid (0.31 g): **NMR (¹H, CDCl₃):** δ 8.32 (bs, 1 H), 8.02 (s, 1 H), 7.10-7.22 (m, 2H), 3.91 (s, 3H), 2.71 (s, 3H). **MS *(m*/*****z)**:* 222 [M-H]⁻(1Cl).

### Preparation 15 (P15): 2-Ethyl-1H-indole-3-carboxylic acid ethyl ester

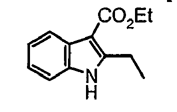

The title compound (5 mmol scale) was obtained by:
1. Procedure G6 but using 3-oxo-valeric acid ethyl ester instead of acetylacetate methyl ester to give the product as a yellow oil (0.90 g).
2. Treating this material as described in Procedure G7 to give the title compound as an off-white solid (0.12 g): **NMR (¹H, CDCl₃):** δ 8.52 (bs, 1 H), 8.15 (s, 1 H), 7.15-7.37 (m, 3H), 4.44 (q, 2H), 3.23 (q, 2H), 1.47 (t, 3H), 1.36 (t, 3H). **MS (*m*/*z*):** 216 [M-H]⁻.

### Preparation 16 (P16): 2-(4-Chloro-2-nitro-phenyl)-3-hydroxy-but-2-enoic acid methyl ester

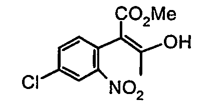

The title compound was obtained as a yellow oil (1.3 g) following Procedure G6 using 5-chloro-2-fluoronitrobenzene (5 mmol). **NMR (¹H, CDCl₃):** δ 7.97 (d, 1 H), 7.56 (dd, 1 H), 7.23 (d, 1 H), 3.62 (s, 3H), 1.82 (s, 3H) (enol-OH not monitored). **MS *(m*/*****z)**:* 270 [M-H]⁻ (1Cl).

### Preparation 17 (P17): 6-Chloro-2-methyl-1H-indole-3-carboxylic acid methyl ester

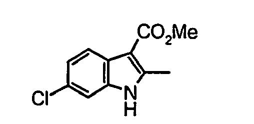

The title compound was obtained as a colourless solid (0.60 g) by treating 2-(4-chloro-2-nitro-phenyl)-3-hydroxy-but-2-enoic acid methyl ester (4.7 mmol) as described in Procedure G7. **NMR (¹H, CDCl₃):** δ 8.25 (bs, 1 H), 7.96 (d, 1 H), 7.12-7.27 (m, 2H), 3.94 (s, 3H), 2.70 (s, 3H). **MS *(m*/*****z)**:* 222 [M-H]⁻ (1Cl).

The following substituted indoles (P18-P20) were prepared in analogy to Preparations 16 and 17 using Procedures G6 followed bv G7.

| | | |
|---|---|---|
| **Preparation 18 (P18): 5- Fluoro-2-methyl-1*H*- indole-3-carboxylic acid methyl ester** | 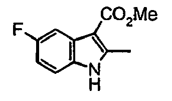 | **NMR (¹H, CDCl₃):** δ 8.30 (bs, 1H), 7.72 (d, 1 H), 7.15-7.22 (m, 1 H), 6.90 (t, 1 H), 3.91 (s, 3H), 2.73 (s, 3H). **MS (*m*/*z*)*:*** 206 [M-H]⁻. |
| **Preparation 19 (P19): 6- Fluoro-2-methyl-1 *H*- indole-3-carboxylic acid methyl ester** | 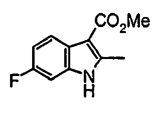 | **NMR (¹H, CDCl₃):** δ 8.30 (bs, 1H), 7.99 (dd, 1 H), 7.90-7.03 (m, 2H), 3.91 (s, 3H), 2.73 (s, 3H). **MS (*m*/*z*)**: 206 [M-H]⁻. |
| **Preparation 20 (P20): 7- Fluoro-2-methyl-1*H*- indole-3-carboxylic acid methyl ester** | 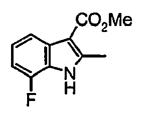 | **NMR (¹H, CDCl₃):** δ 8.46 (bs, 1H), 7.82 (d, 1H), 7.04-7.16 (m, 1 H), 6.89 (t, 1 H), 3.94 (s, 3H), 2.77 (s, 3H). **MS (*m*/*z*):** 206 [M-H]⁻. |

### Preparation 21 (P21): 5-Chloro-1,2-dimethyl-1H-indole-3-carboxylic acid methyl ester

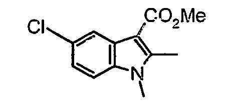

The title compound was obtained as a yellow powder (0.29 g) following Procedure G4 using 5-chloro-2-methyl-1*H*-indole-3-carboxylic acid methyl ester (0.28 g). **NMR (¹H, CDCl₃):** δ 8.05 (s, 1H), 7.05-7.20 (m, 2H), 3.92 (s, 3H), 3.67 (s, 3H), 2.74 (s, 3H). **MS (*m*/*z*):** 238 [M+H]⁺ (1Cl).

The following substituted indoles (P22-P26) were prepared in analogy to Preparation 21 using Procedure G4.

| | | |
|---|---|---|
| **Preparation 22 (P22): 6- Chloro-1,2-dimethyl-1*H*- indole-3-carboxylic acid methyl ester** | 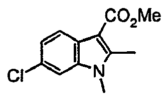 | **NMR (¹H, CDCl₃):** δ 7.98 (d, 1H), 7.26 (d, 1 H), 7.16 (dd, 1H), 3.90 (s, 3H), 3.66 (s, 3H), 2.74 (s, 3H). **MS (*m*/*z*)**: 238 [M+H]⁺ (1 Cl). |
| **Preparation 23 (P23): 2- Ethyl-1-methyl-1*H*- indole-3-carboxylic acid ethyl ester** | 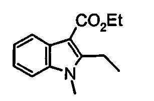 | **NMR (¹H, CDCl₃): δ** 8.08-8.16 (m, 1H), 7.16-7.32 (m, 3H), 4.37 (q, 2H), 3.72 (s, 3H), 3.23 (q, 2H), 1.42 (t, 3H), 1.24 (t, 3H). **MS (*m*/*z*)**: 232 [M+H]⁺. |
| **Preparation 24 (P24): 5- Fluoro-1,2-dimethyl-1*H*- indole-3-carboxylic acid methyl ester** | 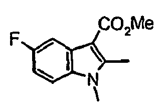 | **NMR (¹H, CDCl₃)**: δ 7.73 (dd, 1 H), 7.18 (dd, 1 H), 6.94 (td, 1 H), 3.89 (s, 3H), 3.68 (s, 3H), 2.74 (s, 3H). **MS (*m*/*z*):** 222 [M+H]⁺. |
| **Preparation 25 (P25): 6- Fluoro-1,2-dimethyl-1*H*- indole-3-carboxylic acid methyl ester** | 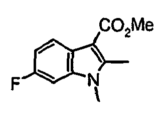 | **NMR (¹H, CDCl₃)**: δ 8.00 (dd, 1 H), 6.91-7.00 (m, 2H), 3.89 (s, 3H), 3.65 (s, 3H), 2.73 (s, 3H). **MS *(m*/*****z)**:* 222 [M+H]⁺. |
| **Preparation 26 (P26): 7- Fluoro-1,2-dimethyl-1*H*- indole-3-carboxylic acid methyl ester** | 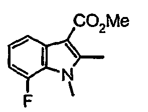 | **NMR (¹H, CDCl₃):** δ 7.84 (d, 1 H), 7.06 (dd, 1H), 6.85 (dd, 1 H), 3.90 (s, 6H), 2.73 (s, 3H). **MS (*m*/*z*)**: 222 [M+H]⁺. |

### Preparation 27 (P27): 5-Chloro-2-chloromethyl-1-methyl-1H-indole-3-carboxylic acid methyl ester

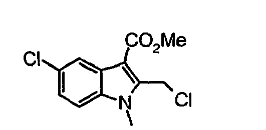

The title compound (0.25 g off-white solid) was obtained from 5-chloro-1,2-dimethyl-1*H-*indole-3-carboxylic acid methyl ester (1.2 mmol) according to Procedure G5: **NMR (¹H, CDCl₃);** δ 8.11 (s, 1 H), 7.11-7.28 (m, 2H), 5.25 (s, 2H), 3.96 (s, 3H), 3.83 (s, 3H).

The following substituted indoles (P28-P32) were prepared in analogy to Preparation 27 using Procedure G5.

| | | |
|---|---|---|
| **Preparation 28 (P28): 6- Chloro-2-chloromethyl- 1-methyl-1*H*-indole-3- carboxylic acid methyl ester** | 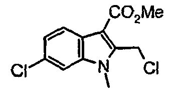 | **NMR (¹H, CDCl₃):** δ 8.04 (d, 1H), 7.34 (s, 1 H), 7.20 (d, 1 H), 5.24 (s, 2H), 3.94 (s, 3H), 3.80 (s, 3H). |
| **Preparation 29 (P29): 2- (1-Chloroethyl)-1- methyl-1*H*-indole-3- carboxylic acid ethyl ester** | 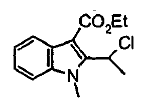 | **NMR (¹H, CDCl₃)**: δ 8.02 (d, 1 H), 7.10-7.28 (m, 3H), 6.80 (q, 1 H), 4.32 (q, 2H), 3.91 (s, 3H), 1.85 (d, 3H), 1.35 (t, 3H). |
| **Preparation 30 (P30): 2- Chloromethyl-5-fluoro-1- methyl-1*H*-indole-3- carboxylic acid methyl ester** | 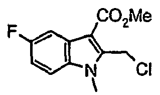 | **NMR (¹H, CDCl₃):** δ 7.78 (dd, 1H), 7.25 (dd, 1 H), 7.03 (td, 1H), 5.26 (s, 2H), 3.95 (s, 3H), 3.83 (s, 3H). |
| **Preparation 31 (P31): 2- Chloromethyl-6-fluoro-1- methyl-1*H*-indole-3- carboxylic acid methyl ester** | 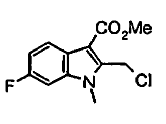 | **NMR (¹H, CDCl₃):** δ 8.06 (dd, 1H), 6.95-7.05 (m, 2H), 5.24 (s, 2H), 3.92 (s, 3H), 3.79 (s, 3H). |
| **Preparation 32 (P32): 2- Chloromethyl-7-fluoro-1- methyl-1*H*-indole-3- carboxylic acid methyl ester** | 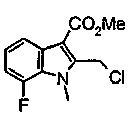 | **NMR (¹H, CDCl₃):** δ 7.89 (d, 1H), 7.10 (td, 1H), 6.94 (dd, 1 H), 5.24 (s, 2H), 4.06 (s, 3H), 3.93 (s, 3H). |

### Preparation 33 (P33): 2-Methyl-benzo[b]thiophene-3-carboxylic acid methyl ester

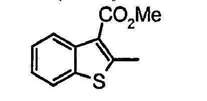

To a stirred suspension of AlCl₃ (0.93 g) in dry 1,2-dichloroethane (2 ml) at 0 °C was added oxalyl chloride (0.61 ml). After 20 min 2-methyl-benzo[*b*]thiophene (0.62 g) in dry 1,2-dichloroethane (1 ml) was added dropwise *via* syringe. After an additional 15 min the mixture was poured onto ice and conc. aqueous HCl (2 ml). This was extracted twice with CH₂Cl₂ . Evaporation of the volatiles *in vacuo* gave a residue which was vigorously stirred with NaOH *(ca.* 1.5 g) in water until a clear solution was obtained. This was washed with Et₂O, acidified using excess HCl and extracted with Et₂O. This extract was washed (brine) and concentrated to give a cream solid (0.6 g). This was dissolved in dry CH₂Cl₂ (15 ml) containing catalytic DMF (2 drops) and at 0 °C was added with stirring oxalyl chloride (0.41 ml). The ice bath was removed. After gas evolution had ceased (2 h) volatiles were removed *in vacuo.* To the residue was added dry methanol (5 ml) and the mixture was kept for 16 h. The material obtained after evaporation of the volatiles was submitted to column chromatography to give the title compound (0.12 g) as a yellow oil. **NMR (¹H**, **CDCl₃)**: δ 8.41 (d, 1 H), 7.75 (t, 1 H), 7.42 (t, 1H), 7.35 (d, 1 H), 3.98 (s, 3H), 2.85 (s, 3H) [n.b. in a major by-product these protons are shifted to 2.75 (s, 3H)].

### Example 1: 6-Bromo-5-methoxy-2-[4-methoxy-3-(2-piperidin-1-yl-ethoxy)phenyl]-8-methyl-1,8-dihydro-2H-2,8-diazacyclopenta[a]inden-3-one hydrochloride

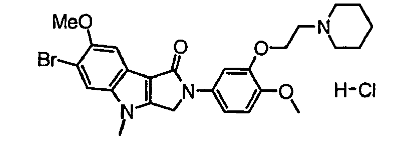

6-Bromo-2-bromomethyl-5-methoxy-2-methyl-1*H*-indole-3-carboxylic acid methyl ester (P6, 29 mg) and 4-methoxy-3-(2-piperidin-1-yl-ethoxy)-phenylamine hydrochloride (P2, 1.1 eq.) were heated in dry DMF (0.5 ml) for 2 h at 120 °C. NaHCO₃ (*ca*. 100 mg) was added and heating continued for additional 2 h. The mixture was concentrated *in vacuo* and the residue submitted to column chromatography. From this was obtained a mixture of compounds (28 mg) that was dissolved tn dry CH₂Cl₂ (1 ml) and allowed to react with Me₃Al (2 M in toluene, 0.08 ml) for 6 h. The solution was partitioned between aqueous NaOH (1 M) and CH₂Cl₂ :iPrOH 3:1. The organic layer was collected, volatiles evaporated and the residue submitted to column chromatography, follwed by conversion to the hydrochloride salt (G1). Trituration with MeOH (1 ml *2) gave the title compound as an off-white solid (6 mg): **NMR (¹H, d4-MeOH):** δ 7.75 (s, 1H), 7.72 (d, 1H), 7.53 (s, 1H), 7.18 (dd, 1 H), 7.09 (d, 1 H), 4.95 (s, 2H), 4.42 (t, 2H), 3.94 (s, 3H), 3.91 (s, 3H), 3.83 (s, 3H), 3.74 (d, 2H), 3.58 (t, 2H), 3.10 (t, 2H), 2.04 (d, 2H), 1.80-1.93 (m, 3H), 1.52-1.63 (m, 1H). **MS (*m*/*z*):** 528/530 [MH]⁺ (1 Br). **m.p.** 227-229 °C.

### Example 2: 5-Methoxy-2-[4-methoxy-3-(2-piperidin-1-yl-ethoxy)phenyl]-8-methyl-1,8-dihydro-2H-2,8-diazacyclopenta[a]inden-3-one hydrochloride

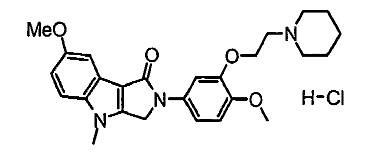

6-Bromo-5-methoxy-2-[4-methoxy-3-(2-piperidin-1-yl-ethoxy)phenyl]-8-methyl-1,8-dihydro-2*H*-2,8-diazacyclopenta[a]inden-3-one (38 mg) in EtOAc (5 ml) and EtOH (*ca*. 0.2 ml) was hydrogenated at atmospheric pressure on Pd/C (10%, 10 mg) for 16 h and on Pd/C (10%, 100 mg) for 8 h. The mixture was filtered through Celite, volatiles evaporated and the residue submitted to column chromatography followed by preparative reverse phase HPLC, follwed by conversion to the hydrochloride salt (G1) to give the product as an orange gum (2 mg). **NMR (¹H, d4-MeOH)**: δ 7.64 (d, 1 H), 7.28-7.35 (m, 2H), 7.09 (d, 1 H), 6.99 (d, 1 H), 6.85 (d, 1 H), 4.86 (s, 2H), 4.32 (t, 2H), 3.80 (s, 3H), 3.75 (s, 3H), 3.72 (s, 3H), 3.58-3.68 (m, 2H), 3.49 (t, 2H), 2.93-3.08 (m, 2H), 1.84-1.99 (m, 2H), 1.63-1.84 (m, 3H), 1.40-1.60 (m, 1H). **MS (*m*/*z*)*:*** 450 [MH]⁺.

### Example 3: 5,6-Dichloro-2-[4-methoxy-3-(2-piperidin-1-yl-ethoxy)phenyl]-8-methyl-1,8-dihydro-2H-2,8-diazacyclopenta[a]inden-3-one hydrochloride

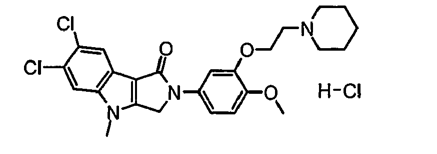

2-Chloromethyl-5,6-dichloro-1-methyl-1*H*-indole-3-carboxylic acid *tert*-butyl ester (P13, 91 mg) and 4-methoxy-3-(2-piperidin-1-yl-ethoxy)-phenylamine hydrochloride (P2, 75 mg) were heated in dry DMF (0.7 ml) and dry dioxane (0.5 ml) for 2 h at 100 °C. The mixture was concentrated *in vacuo* and the residue submitted to column chromatography. From this was obtained an *N*-alkylation product which was exposed to trifluoroacetic acid (2 ml) in CH₂Cl₂ (2 ml) for 3 h. The volatiles were evaporated *in vacuo* and the residue heated in HCl/dioxane (4 M, *ca*. 30 min). Again, volatiles were evaporated *in vacuo* and to the residue in dry CH₂Cl₂ (*ca*. 1 ml) containing catalytic DMF (*ca*. 0.1 eq.) at 0 °C was added with stirring oxalyl chloride (9 µl). The ice bath was removed and after 2 h volatiles were evaporateded *in vacuo.* The residue was treated with NEt₃ in dioxane and subsequently partitioned between aqueous NaOH (*ca.* 0.5 M) and EtOAc. The organic layer was collected, volatiles evaporated and the residue submitted to column chromatography, follwed by conversion to the hydrochloride salt (G1) to give the title compound as an off-white solid (5 mg): **NMR (¹H, d4-MeOH):** δ 7.99 (s, 1H), 7.70 (d, 1H), 7.18 (dd, 1H), 7.09 (d, 1H), 6.80 (s, 1 H), 4.99 (s, 2H), 4.40 (t, 2H), 3.90 (s) and 3.85 (s, 6H), 3.70-3.76 (m, 2H), 3.57 (t, 2H), 3.05-3.15 (m, 2H), 1.50-1.63 and 1.66-2.05 (m, 5H). **MS (*m*/*z*)*:*** 488 [MH]⁺ (2Cl).

### Example 4: 5-Chloro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride

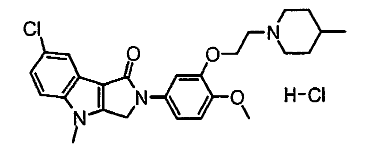

5-Chloro-2-chloromethyl-1-methyl-1*H*-indole-3-carboxylic acid methyl ester (P27, 0.46 mmol) and 4-methoxy-3-[2-(4-methyl-piperidin-1-yl)-ethoxy]-phenylamine hydrochloride (P5, 1.2 eq.) were heated in dry DMF (0.55 ml) for 5 h at 100 °C. Volatiles were evaporated *in vacuo* and the residue submitted to column chromatography. From this was obtained an *N*-alkylation product (38%) a solution of which in CH₂Cl₂ (0.05 M) was allowed to react with Me₃Al (2 M in hexanes, 3 eq.) for 5 h. Silica gel, water (few drops), 10% conc. aqueous NH₃/MeOH:CH₂Cl₂ 1:10 were added with vigorous stirring, the resulting slurry removed by filtration and washed well with 10% conc. aqueous NH₃/MeOH:CH₂Cl₂ 1:10. The solution thus obtained was concentrated *in vacuo* and the residue submitted to column chromatography, follwed by conversion to the hydrochloride salt (G1) to give the title compound as an off-white solid (55 mg): **NMR (¹H, d4-MeOH):** δ 7.86 (d, 1 H), 7.72 (d, 1H), 7.55 (d, 1H), 7.32 (dd, 1 H), 7.23 (dd, 1 H), 7.11 (d, 1 H), 4.97 (s, 2H), 4.45 (t, 2H), 3.92 (s, 3H), 3.88 (s, 3H), 3.80 (d, 2H), 3.62 (t, 2H), 3.18 (t, 2H), 2.01 (d, 2H), 1.78 (m, 1 H), 1.56 (m, 2H), 1.08 (d, 3H) (only data for the major isomer resulting from protonation reported). **MS *(m*/*****z)**:* 468 [MH]⁺ (1Cl).

### Example 5: 2-[4-Methoxy-3-(2-piperidin-1-yl-ethoxy)phenyl]-1,2-dihydro-8-thia-2-azacyclopenta[a]inden-3-one hydrochloride

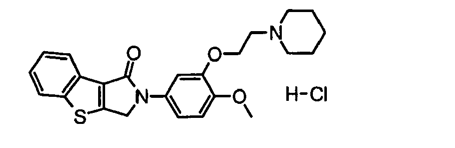

2-Methyl-benzo[*b*]thiophene-3-carboxylic acid methyl ester (P33) was treated as described in G5 to give material (0.17 g) which contained *ca.* 35% of the chloromethyl derivative. Half of this material and 4-methoxy-3-(2-piperidin-1-yl-ethoxy)-phenylamine hydrochloride (P2, 0.45 mmol) were heated in dry DMF (0.5 ml) for 5 h at 100 °C. The mixture was concentrated *in vacuo* and the residue submitted to column chromatography, follwed by conversion to the hydrochloride salt (G1) to give the title compound as an orange film (31 mg). **NMR (¹H, d4-MeOH):** δ 8.24 (d, 1H), 8.04 (d, 1 H), 7.75 (d, 1H), 7.46-7.58 (m, 2H), 7.29 (dd, 1H), 7.15 (d, 1H), 5.16 (s, 2H), 4.46 (t, 2H), 3.94 (s, 3H), 3.78 (d, 2H), 3.61 (t, 2H), 3.14 (t, 2H), 2.02 (d, 2H), 1.80-1.96 (m, 3H), 1.53-1.66 (m, 1 H). **MS *(m*/*****z)**:* 423 [MH]⁺.

### Example 6: 2-{4-Methoxy-3-[2-(4-methyl-piperidin-1-yl)-ethoxy]-phenyl}-1,2-dihydro-8-thia-2-aza-cyclopenta[a]inden-3-one hydrochloride

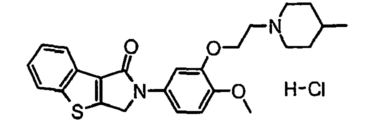

The title compound was obtained as described for Example 5 but using 4-methoxy-3-[2-(4-methyl-piperidin-1-yl)-ethoxy]-phenylamine hydrochloride (P5) to give the product as an orange film (35 mg). **NMR (¹H, d4-MeOH):** δ 8.24 (d, 1H), 8.04 (d, 1H), 7.75 (d, 1H), 7.46-7.58 (m, 2H), 7.29 (dd, 1H), 7.15 (d, 1H), 5.16 (s, 2H), 4.46 (t, 2H), 3.94 (s, 3H), 3.78 (d, 2H), 3.61 (t, 2H), 3.14 (t, 2H), 2.00 (d, 2H), 1.70-1.82 (m, 1H), 1.54 (dq, 2H), 1.08 (d, 3H). **MS *(m*/*z):*** 437 [MH]⁺.

The following examples were prepared in analogy to Example 4 from the respective substituted 2-chloromethyl indoles and the respectlive substituted phenylamine hydrochlorides (P2 respectively P5):

| | | |
|---|---|---|
| **Example 7: 2-[4-Methoxy-3- (2-piperidin-1-yl- ethoxy)phenyl]-8-methyl- 1,8-dihydro-2*H*-2,8- diazacyclopenta[*a*]inden-3- one hydrochloride** | 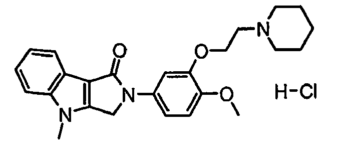 | **NMR (¹H, d4-MeOH):** δ 7.88 (d, 1H), 7.74 (d, 1H), 7.56 (d, 1H), 7.34 (dt, 1H), 7.28 (dt, 1H), 7.21 (dd, 1H), 7.11 (d, 1H), 4.99 (s, 2H), 4.29 (t, 2H), 3.91 (s, 3H), 3.88 (s, 3H), 3.60 (t, 2H), 3.72-3.78 (m, 2H), 3.08-3.15 (d, 2H), 1.97- 2.05 (m, 2H), 1.80-1.93 (m, 3H), 1.53-1.62 (m, 1H). **MS (*m*/*z*):** 420 [MH]⁺. |
| **Example 8: 5-Chloro-2-[4- methoxy-3-(2-piperidin-1- yl-ethoxy)phenyl]-8- methyl-1,8-dihydro-2*H*-2,8- diazacyclopenta[a]inden-3- one hydrochloride** | 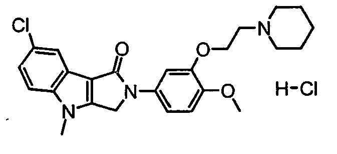 | **NMR (¹H, d4-MeOH):** δ 7.86 (d, 1H), 7.72 (d, 1H), 7.55 (d, 1H), 7.32 (dd, 1H), 7.23 (dd, 1H), 7.11 (d, 1H), 4.98 (s, 2H), 4.45 (t, 2H), 3.92 (s, 3H), 3.88 (s, 3H), 3.77 (d, 2H), 3.62 (t, 2H), 3.14 (t, 2H), 2.01 (d, 2H), 1.80-1.95 (m, 3H), 1.54-1.68 (m, 1 H). **MS *(m*/*****z)**:* 454 [MH]⁺ (1Cl). |
| **Example 9: 6-Chloro-2-[4- methoxy-3-(2-piperidin-1- yl-ethoxy)phenyl]-8- methyl-1,8-dihydro-2*H*-2,8- diazacyclopenta[a]inden-3- one hydrochloride** | 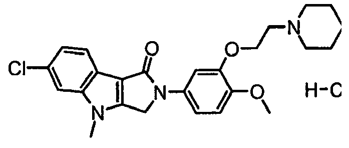 | **NMR (¹H, d4-MeOH):** δ 7.85 (d, 1H), 7.74 (d, 1H), 7.64 (d, 1H), 7.28 (dd, 1H), 7.22 (dd, 1H), 7.12 (d, 1H), 5.01 (s, 2H), 4.45 (t, 2H), 3.92 (s, 3H), 3.88 (s, 3H), 3.77 (d, 2H), 3.62 (t, 2H), 3.14 (t, 2H), 2.01 (d, 2H), 1.80-1.95 (m, 3H), 1.54-1.68 (m, 1H). **MS (*m*/*z*):** 454 [MH]⁺ (1Cl). |
| **Example 10: 6-Fluoro-2-[4- methoxy-3-(2-piperidin-1- yl-ethoxy)phenyl]-8- methyl-1,8-dihydro-2*H*-2,8- diazacyclopenta[a]inden-3- one hydrochloride** | 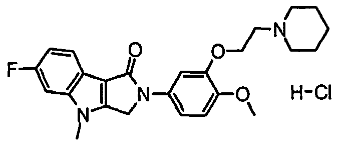 | **NMR (¹H, d4-MeOH):** δ 7.83 (m, 1H), 7.68 (d, 1H), 7.32 (dd, 1H), 7.20 (dd, 1H), 6.88-7.00 (m, 2H), 4.94 (s, 2H), 4.30 (t, 2H), 3.91 (s, 3H), 3.83 (s, 3H), 3.76 (d, 2H), 3.59 (t, 2H), 3.13 (t, 2H), 1.95 (d, 2H), 1.65-1.83 (m, 3H), 1.37-1.53 (m, 1H). |
| **Example 11: 2-{4-Methoxy- 3-[2-(4-methyl-piperidin-1- yl)-ethoxy]-phenyl}-1,8- dimethyl-1,8-dihydro-2*H*- 2,8- diazacyclopenta[a]inden-3- one hydrochloride** | 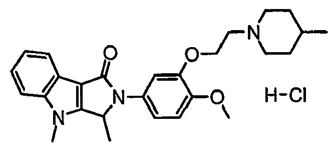 | **NMR (¹H, d4-MeOH):** δ 7.86 (d, 1H), 7.54 (d, 1H), 7.35 (d, 1H), 7.33 (t, 1H), 7.25 (t,1H), 7.13 (d, 1H), 7.07 (d, 1H), 5.42 (q, 1H), 4.35-4.45 (m, 2H), 3.90 (s, 6H), 3.74-3.80 (m, 2H), 3.55-3.61 (m, 2H), 3.11 (t, 2H), 1.88-2.03 (m, 2H), 1.65-1.83 (m, 1H), 1.42-1.58 (m, 5H), 1.04 (d, 3H) (only data for the major isomer resulting from protonation reported). **MS *(m*/*****z)**:* 448 [MH]⁺. |
| **Example 12: 2-{4-Methoxy- 3-[2-(4-methyl-piperidin-1 - yl)-ethoxy]-phenyl}-8- methyl-1,8-dihydro-2*H*-2,8- diazacyclopenta[a] inden-3- one hydrochloride** | 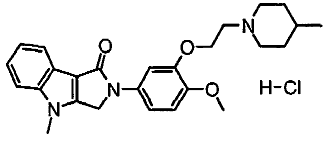 | **NMR (¹H, d4-MeOH):** δ 7.83 (d, 1H), 7.51 (d, 1H), 7.30 (dt, 1H), 7.22 (dt, 1H), 7.16 (dd,1H), 7.10 (d, 1H), 7.06 (d, 1H), 4.95 (s, 2H), 4.38 (t, 2H), 3.87 (s, 3H), 3.85 (s, 3H), 3.74 (d, 2H), 3.54 (t, 2H), 3.08 (dt, 2H), 1.96 (bd, 2H), 1.65-1.80 (m, 1 H), 1.48 (dq, 2H), 1.02 (d, 3H) (only data for the major isomer resulting from protonation reported). **MS (*m*/*z*):** 434 [MH]⁺. |
| **Example 13: 6-Chloro-2-{4- methoxy-3-[2-(4-methyl- piperidin-1-yl)-ethoxy]- phenyl}-8-methyl-1,8- dihydro-2*H*-2,8- diazacyclopenta[*a*]inden-3- one hydrochloride** | 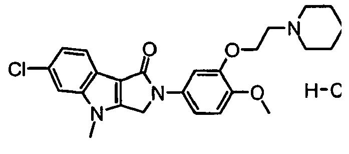 | **NMR (¹H, d4-MeOH):** δ 7.84 (d, 1H), 7.72 (d, 1 H), 7.63 (d, 1H), 7.28 (dd, 1H), 7.22 (dd, 1H), 7.12 (d, 1H), 4.98 (s, 2H), 4.44 (t, 2H), 3.92 (s, 3H), 3.87 (s, 3H), 3.77 (d, 2H), 3.62 (t, 2H), 3.14 (t, 2H), 2.01 (d, 2H), 1.70-1.84 (m, 1 H), 1.55 (dq, 2H), 1.04 (d, 3H) (only data for the major isomer resulting from protonation reported). **MS (*m*/*z*):** 468 [MH]⁺. |
| **Example 14: 5-Fluoro-2-{4- methoxy-3-[2-(4-methyl- piperidin-1-yl)-ethoxy]- phenyl}-8-methyl-1,8- dihydro-2*H*-2,8- diazacyclopenta[a]inden-3- one hydrochloride** | 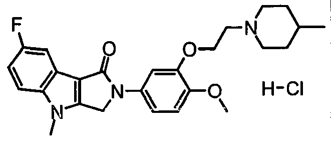 | **NMR (¹H, d4-MeOH):** δ 7.73 (d, 1H), 7.54-7.57 (m, 2H), 7.18 (dd, 1H), 7.08-7.13 (m, 2H), 4.99 (s, 2H), 4.43 (t, 2H), 3.91 (s, 3H), 3.88 (s, 3H), 3.76 (d, 2H), 3.58 (t, 2H), 3.12 (t, 2H), 2.01 (d, 2H), 1.66-1.82 (m, 1 H), 1.46-1.58 (m, 2H), 1.06 (d, 3H) (only data for the major isomer resulting from protonation reported). |
| **Example 15: 6-Fluoro-2-{4- methoxy-3-[2-(4-methyl- piperidin-1-yl)-ethoxy]- phenyl}-8-methyl-1,8- dihydro-2*H*-2,8- diazacyclopenta[a]inden-3- one hydrochloride** | 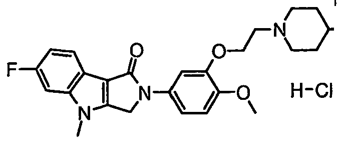 | **NMR (¹H, d4-MeOH):** δ 7.83 (m, 1H), 7.68 (d, 1H), 7.32 (dd, 1H), 7.20 (dd, 1H), 6.87-6.98 (m, 2H), 4.94 (s, 2H), 4.43 (t, 2H), 3.91 (s, 3H), 3.83 (s, 3H), 3.76 (d, 2H), 3.59 (t, 2H), 3.13 (t, 2H), 2.01 (d, 2H), 1.53-1.73 (m, 1H), 1.33-1.53 (m, 2H), 1.06 (d, 3H) (only data for the major isomer resulting from protonation reported). |
| **Example 16: 7-Fluoro-2-{4- methoxy-3-[2-(4-methyl- piperidin-1-yl)-ethoxy]- phenyl}-8-methyl-1,8- dihydro-2*H*-2,8- diazacyclopenta[a]inden-3- one hydrochloride** | 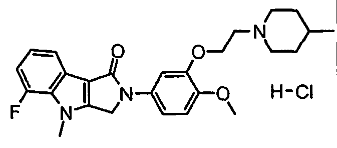 | **NMR (¹H, d4-MeOH):** δ 7.75 (dd, 1 H), 7.68 (d, 1 H), 6.90-7.15 (m, 4H), 4.94 (s, 2H), 4.43 (t, 2H), 3.95 (s, 3H), 3.80 (s, 3H), 3.76 (d, 2H), 3.59 (t, 2H), 3.05 (t, 2H), 1.99 (d, 2H), 1.55- 1.75 (m, 1 H), 1.32-1.50 (m, 2H), 1.06 (d, 3H) (only data for the major isomer resulting from protonation reported). |

## Claims

1. A compound of formula (I) or a pharmaceutically acceptable salt thereof: 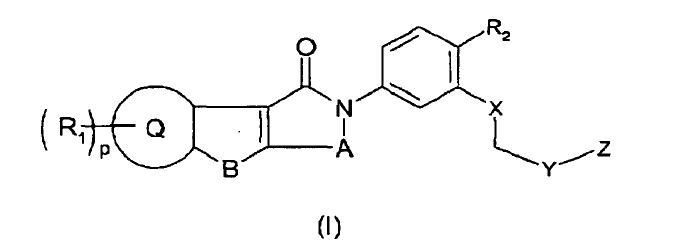 wherein:
Q is phenyl or a 6-membered heteroaromatic group containing at least one nitrogen atom;
A is -(CH₂-CH₂)-, -(CH=CH)-, -(CH₂)₃-, -C(CH₃)₂-, -(CH=CH-CH₂)-, or a group - (CHR₃)- wherein R₃ is hydrogen, halogen, hydroxy, cyano, nitro, C₁₋₆alkyl, C₃₋₇ cycloalkyl, C₃₋₇cycloalkyloxy, haloC₁₋₆alkyl, C₁₋₆alkoxy, haloC₁₋₆alkoxy or C₁₋₆alkylthio;
B is O, S or NR₁₁, wherein R₁₁ is hydrogen or C₁₋₆alkyl or C₁₋₆alkanoyl which may be substitued by one or more C₁₋₆alkoxy;
R₁ is halogen, cyano, C₁₋₆alkyl, C₃₋₇cycloalkyl, C₃₋₇cycloalkyloxy, C₁₋₆alkoxy, C₁₋₆alkylthio, hydroxy, amino, mono- or di-C₁₋₆alkylamino, an N-linked 4 to 7 membered heterocyclic group, nitro, haloC₁₋₆alkyl, haloC₁₋₆alkoxy, aryl, arylC₁₋₆alkyl, arylC₁₋₆alkyloxy, arylC₁₋₆alkylthio or COOR₄ or COR₅ wherein R₄. and R₅ are independently hydrogen or C₁₋₆alkyl or COR₆ wherein R₆ is amino, mono- or di-C₁₋₆alkylamino or an N-linked 4 to 7 membered heterocyclic group;
p is 0, 1 or 2 or 3;
R₂ is hydrogen, halogen, hydroxy, cyano, nitro, C₁₋₆alkyl, C₁₋₆alkanoyl, C₃₋₇ cycloalkyl, C₃₋₇cycloalkyloxy, haloC₁₋₆alkyl, C₁₋₆alkoxy, haloC₁₋₆alkoxy, C₁₋₆alkylthio, amino, mono- or di-C₁₋₆alkylamino or an N-linked 4 to 7 membered heterocyclic group;
X is oxygen, sulfur, -CH₂- or NR₈ wherein R₈ is hydrogen or C₁₋₆alkyl;
Y is a single bond, -CH₂-, -(CH₂)₂- or -CH=CH-; and
Z is a N-linked heterocyclic group or a C-linked 4 to 7 membered heterocyclic group containing at least one nitrogen, which may be substituted by one or more C₁₋₆ alkyl, amino, mono- or di-C₁₋₆alkylamino, a heteroaromatic group, an aromatic or non-aromatic N-linked or C-linked heterocycle or an aromatic or non aromatic heterocycle C₁₋₆alkyl, that may be substituted by one or more C₁₋₆alkyl;
or Z is -NR₉R₁₀ where R₉ and R₁₀ are independently hydrogen or C₁₋₆alkyl.

2. A compound as claimed in claim 1, wherein A is -CH₂-.

3. A compound as claimed in claim 1 or 2, wherein p is 1 or 2 and R₁ is/are halogen, particularly chloro or fluoro.

4. A compound as claimed in claim 1, 2 or 3, wherein R₂ is C₁₋₆alkoxy (particularly methoxy), halogen or cyano.

5. A compound as claimed in any of claims 1-4, wherein X is oxygen.

6. A compound as claimed in any of claims 1-5, wherein Y is -CH₂-.

7. A compound as claimed in any of claims 1-6, wherein Z is an optionally substituted N-linked 4 to 7 membered heterocycle, in particular piperidyl.

8. A compound as claimed in claim 1 having the formula (Ia): 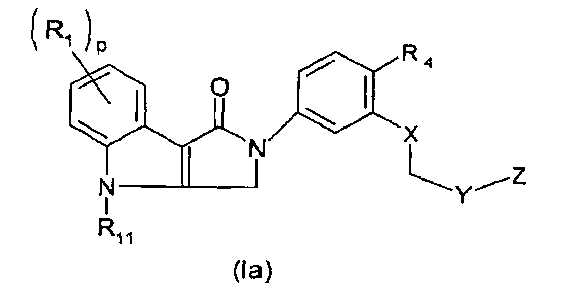 wherein R₁, p, R₁₁, R₄, X, Y and Z, are as defined In claim 1.

9. A compound as claimed in claim 1 which is:
6-Bromo-5-methoxy-2-[4-methoxy-3-(2-piperidin-1-yl-ethoxy)phenyl]-8-methyl-1,8-dihydro-2H-2,8-diazacyclopenta[a]inden-3-one hydrochloride
5-Methoxy-2-[4-methoxy-3-(2-piperidin-1-yl-ethoxy)phenyl]-8-methyl-1,8-dihydro-2H-2,8-diazacyclopenta[a]inden-3-one hydrochloride
5,6-Dichloro-2-[4-methoxy-3-(2-piperidin-1-yl-ethoxy)phenyl]-8-methyl-1,8-dihydro-2H-2,8-diazacyclopenta[a]inden-3-one hydrochloride
5-Chloro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
2-[4-Methoxy-3-(2-piperidin-1-yl-ethoxy)phenyl]-1,2-dihydro-8-thia-2-azacyclopenta[a]inden-3-one hydrochloride
2-{4-Methoxy-3-[2-(4-methyl-piperidin-1-yl)-ethoxy]-phenyl}-1,2-dihydro-8-thia-2-azacyclopenta[a]inden-3-one hydrochloride
2-[4-Methoxy-3-(2-piperidin-1-yl-ethoxy)phenyl]-8-methyl-1,8-dihydro-2H-2,8-diazacyclopenta[a]inden-3-one hydrochloride
5-Chloro-2-[4-methoxy-3-(2-piperidin-1-yl-ethoxy)phenyl]-8-methyl-1,8-dihydro-2H-2,8-diazacyclopenta[a]inden-3-one hydrochloride
6-Chloro-2-[4-methoxy-3-(2-piperidin-1-yl-ethoxy)phenyl]-8-methyl-1,8-dihydro-2H-2,8-diazacyclopente[a]inden-3-one hydrochloride
6-Fluoro-2-[4-methoxy-3-(2-piperidin-1-yl-ethoxy)phenyl]-8-methyl-1,8-dihydro-2H-2,8-diazacyclopenta[a]inden-3-one hydrochloride
2-{4-Methoxy-3-[2-(4-methyl-piperidin-1-yl)-ethoxy]-phenyl}-1,8-dimethyl-1,8-dihydro-2H-2,8-diazacyclopenta[a]inden-3-one hydrochloride
2-{4-Methoxy-3-[2-(4-methyl-piperidin-1-yl)-ethoxy]-phenyl}-8-methyl-1,8-dihydro-2H-2,8-diazacyclopenta[a]inden-3-ons hydrochloride
6-Chloro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
5-Fluoro-2-[4-methoxy-3-{2-(4-methyl-piperidin-1-yl)-ethoxy}-phenyl]-8-methyl-1,8-dihydro-2H-2,8-diazacyclopenta[a]inden-3-one hydrochloride
6-Fluoro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
7-Fluoro-2-{4-methoxy-3-[2-(4-methyl-piperidin-1-yl)-ethoxy]-phenyl}-8-methyl-1,8-dihydro-2H-2,8-diazacyclopenta[a]inden-3-one hydrochloride
or a pharmaceutically acceptable salt thereof.

10. A process for the preparation of a compound as claimed in claim 1, which process comprises:
(a) reacting a compound of formula (II): 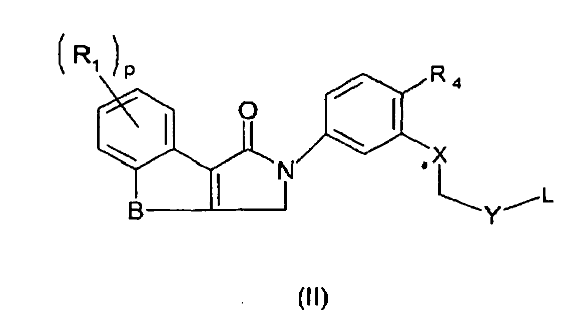 wherein R₁, R₂, p, A, X, and Y are as defined for formula (I), and L is a leaving group, with a compound of formula (III):
Z-H (III)
wherein Z is as defined for formula (I); or
(b) reacting a compound of formula (III): 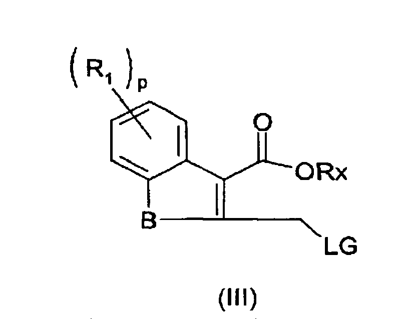 with a compound of formula (IV) or a salt thereof: 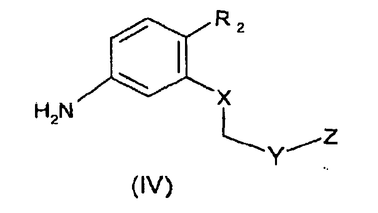 followed, as appropriate, with a treatment of the intermediate obtained with AlMe₃ or a similar oxophilic reagent;
and for either process (a) or process (b), optionally followed by:
removing any protecting groups; and/or
converting a compound of formula (I) into another compound of formula (I); and/or forming a pharmaceutically acceptable salt.

11. A pharmaceutical composition comprising a compound as defined in any of claims 1-9 and a pharmaceutically acceptable carrier or excipient.

12. A process for preparing a pharmaceutical composition, the process comprising mixing a compound as claimed in any of claims 1-9 and a pharmaceutically acceptable carrier or excipient.

13. A compound as claimed in any of claims 1-9 for use as a therapeutic substance.

14. A compound as claimed in any of claims 1-9 for use in the treatment of a CNS disorder.

15. A compound as claimed in claim 14, wherein the disorder is depression or anxiety.

16. Use of a compound as claimed in any of claims 1-9 in the manufacture of a medicament for use in the treatment of a CNS disorder.

17. The use as claimed in claim 16, wherein the disorder is depression or anxiety.

## Patentansprüche

1. Verbindung der Formel (I) oder ein pharmazeutisch verträgliches Salz davon: 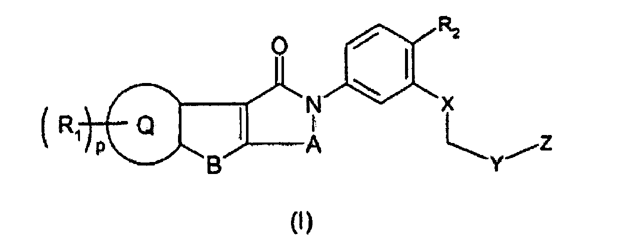 wobei:
Q Phenyl oder ein 6-gliedriger heteroaromatischer Rest ist, der mindestens ein Stickstoffatom enthält;
A gleich -(CH₂-CH₂)-, -(CH=CH)-, -(CH₂)₃-, -C(CH₃)₂-, -(CH=CH-CH₂)- oder ein Rest -(CHR₃)- ist, wobei R₃ Wasserstoff, Halogen, Hydroxy, Cyano, Nitro, C₁₋₆-Alkyl, C₃₋₇-Cycloalkyl, C₃₋₇-Cycloalkyloxy, Halogen-C₁₋₆-alkyl, C₁₋₆-Alkoxy, Halogen-C₁₋₆-alkoxy oder C₁₋₆-Alkylthio ist;
B gleich O, S oder NR₁₁ ist, wobei R₁₁ Wasserstoff oder C₁₋₆-Alkyl oder C₁₋₆-Alkanoyl ist, das mit einem öder mehreren C₁₋₆-Alkoxyresten substituiert sein kann;
R₁ Halogen, Cyano, C₁₋₆-Alkyl, C₃₋₇-Cycloalkyl, C₃₋₇-Cycloalkyloxy, C₁₋₆-Alkoxy, C₁₋₆-Alkylthio, Hydroxy, Amino, Mono- oder Di-C₁₋₆-alkylamino, ein N-gebundener 4- bis 7-gliedriger heterocyclischer Rest, Nitro, Halogen-C₁₋₆-alkyl, Halogen-C₁₋₆-alkoxy, Aryl, Aryl-C₁₋₆-alkyl, Aryl-C₁₋₆-alkyloxy, Aryl-C₁₋₆-alkylthio oder COOR₄ oder COR₅, wobei R₄ und R₅ unabhängig voneinander Wasserstoff oder C₁₋₆-Alkyl sind, oder COR₆ ist, wobei R₆ Amino, Mono- oder Di-C₁₋₆-alkylamino oder ein N-gebundener 4- bis 7-gliedriger heterocyclischer Rest ist;
p 0, 1 oder 2 oder 3 ist;
R₂ Wasserstoff, Halogen, Hydroxy, Cyano, Nitro, C₁₋₆-Alkyl, C₁₋₆-Alkanoyl, C₃₋₇-Cycloalkyl, C₃₋₇-Cycloalkyloxy, Halogen-C₁₋₆-alkyl, C₁₋₆-Alkoxy, Halogen-C₁₋₆-alkoxy, C₁₋₆-Alkylthio, Amino, Mono- oder Di-C₁₋₆-alkylamino oder ein N-gebundener 4- bis 7-gliedriger heterocyclischer Rest ist;
X Sauerstoff, Schwefel, -CH₂- oder NR₈ ist, wobei R₈ Wasserstoff oder C₁₋₆-Alkyl ist;
Y eine Einfachbindung, -CH₂-, -(CH₂)₂- oder -CH=CH- ist; und
Z ein N-gebundener heterocyclischer Rest oder ein C-gebundener 4- bis 7-gliedriger heterocyclischer Rest, der mindestens ein Stickstoffatom enthält, welcher mit einem oder mehreren C₁₋₆-Alkyl-, Amino-, Mono- oder Di-C₁₋₆-alkylaminoresten substituiert sein kann, ein heteroaromatischer Rest, ein aromatischer oder nicht-aromatischer N-gebundener oder C-gebundener Heterocyclus oder ein aromatisches oder nicht-aromatischer Heterocyclus-C₁₋₆-alkylrest, der mit einem oder mehreren C₁₋₆-Alkylresten substituiert sein kann, ist;
oder Z gleich -NR₉R₁₀ ist, wobei R₉ und R₁₀ unabhängig voneinander Wasserstoff oder C₁₋₆-Alkyl sind.

2. Verbindung nach Anspruch 1, wobei A gleich -CH₂- ist.

3. Verbindung nach Anspruch 1 oder 2, wobei p 1 oder 2 ist und R₁ Halogen, insbesondere Chlor oder Fluor, ist/sind.

4. Verbindung nach Anspruch 1, 2 oder 3, wobei R₂ C₁₋₆-Alkoxy (insbesondere Methoxy), Halogen oder Cyano ist.

5. Verbindung nach einem der Ansprüche 1 bis 4, wobei X Sauerstoff ist.

6. Verbindung nach einem der Ansprüche 1 bis 5, wobei Y gleich -CH₂- ist.

7. Verbindung nach einem der Ansprüche 1 bis 6, wobei Z ein gegebenenfalls substituierter N-gebundener 4- bis 7-gliedriger Heterocyclus, insbesondere Piperidyl, ist.

8. Verbindung nach Anspruch 1 mit der Formel (Ia): 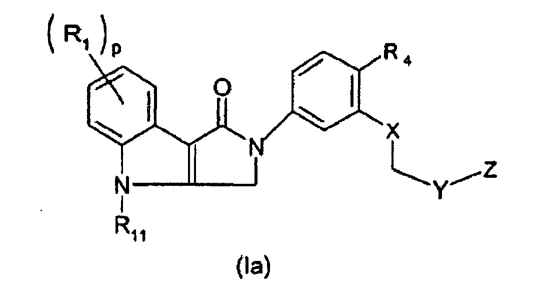 wobei R₁, p, R₁₁, R₄, X, Y und Z wie in Anspruch 1 definiert sind.

9. Verbindung nach Anspruch 1, bei welcher es sich um folgende handelt:
6-Brom-5-methoxy-2-[4-methoxy-3-(2-piperidin-1-yl-ethoxy)phenyl]-8-methyl-1,8-dihydro-2H-2,8-diazacyclopenta[a]inden-3-on-hydrochlorid;
5-Methoxy-2-[4-methoxy-3-(2-piperidin-1-yl-ethoxy)phenyl]-8-methyl-1,8-dihydro-2H-2,8-diazacyclopenta[a]inden-3-on-hydrochlorid;
5,6-Dichlor-2-[4-methoxy-3-(2-piperidin-1-yl-ethoxy)phenyl]-8-methyl-1,8-dihydro-2H-2,8-diazacyclopenta[a]inden-3-on-hydrochlorid;
5-Chlor-2-{4-methoxy-3-[2-(4-methyl-piperidin-1-yl)-ethoxy]-phenyl}-8-methyl-1,8-dihydro-2H-2,8-diazacyclopenta[a]inden-3-on-hydrochlorid;
2-[4-Methoxy-3-(2-piperidin-1-yl-ethoxy)phenyl]-1,2-dihydro-8-thia-2-azacyclopenta[a]inden-3-on-hydrochlorid;
2- {4-Methoxy-3-[2-(4-methyl-piperidin-1-yl)-ethoxy]-phenyl} 1,2-dihydro-8-thia-2-azacyclopenta[a]inden-3-on-hydrochlorid;
2-[4-Methoxy-3-(2-piperidin-1-yl-ethoxy)phenyl]-8-methyl-1,8-dihydro-2H-2,8-diazacyclopenta[a]inden-3-on-hydrochlorid;
5-Chlor-2-[4-methoxy-3-(2-piperidin-1-yl-ethoxy)phenyl]-8-methyl-1,8-dihydro-2H-2,8-diazacyclopenta[a]inden-3-on-hydrochlorid;
6-Chlor-2-[4-methoxy-3-(2-piperidin-1-yl-ethoxy)phenyl]-8-methyl-1,8-dihydro-2H-2,8-diazacyclopenta[a]inden-3-on-hydrochlorid;
6-Fluor-2-[4-methoxy-3-(2-piperidin-1-yl-ethoxy)phenyl]-8-methyl-1,8-dihydro-2H-2,8-diazacyclopenta[a]inden-3-on-hydrochlorid;
2- {4-Methoxy-3-[2-(4-methyl-piperidin-1-yl)-ethoxy]-phenyl} -1,8-dimethyl-1,8-dihydro-2H-2,8-diazacyclopenta[a]inden-3-on-hydrochlorid;
2- {4-Methoxy-3-[2-(4-methyl-piperidin-1-yl)-ethoxy]-phenyl} -8-methyl-1,8-dihydro-2H-2,8-diazacyclopenta[a]inden-3-on-hydrochlorid;
6-Chlor-2- {4-methoxy-3-[2-(4-methyl-piperidin-1-yl)-ethoxy]-phenyl} -8-methyl-1,8-dihydro-2H-2,8-diazacyclopenta[a]inden-3-on-hydrochlorid;
5-Fluor-2- {4-methoxy-3-[2-(4-methyl-piperidin-1-yl)-ethoxy]-phenyl} -8-methyl-1, 8-dihydro-2H-2,8-diazacyclopenta[a]inden-3-on-hydrochlorid;
6-Fluor-2- {4-methoxy-3-[2-(4-methyl-piperidin-1-yl)-ethoxy]-phenyl}-8-methyl-1,8-dihydro-2H-2,8-diazacyclopenta[a]inden-3-on-hydrochlorid;
7-Fluor-2-{4-methoxy-3-[2-(4-methyl-piperidin-1-yl)-ethoxy]-phenyl}-8-methyl-1,8-dihydro-2H-2,8-diazacyclopenta[a]inden-3-on-hydrochlorid;
oder ein pharmazeutisch verträgliches Salz davon.

10. Verfahren zur Herstellung einer Verbindung nach Anspruch 1, wobei das Verfahren umfasst:
(a) Umsetzen einer Verbindung der Formel (II): 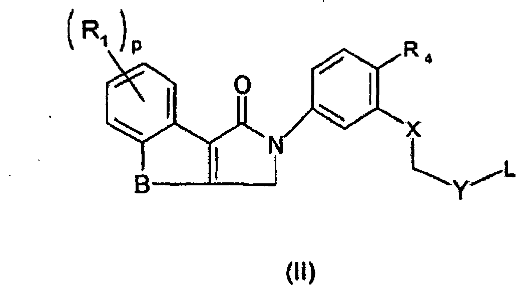 wobei R₁, R₂, p, A, X und Y wie für Formel (I) definiert sind und L eine
Abgangsgruppe ist, mit einer Verbindung der Formel (III):
Z-H (III)
wobei Z wie für Formel (I) definiert ist; oder
(b) Umsetzen einer Verbindung der Formel (III): 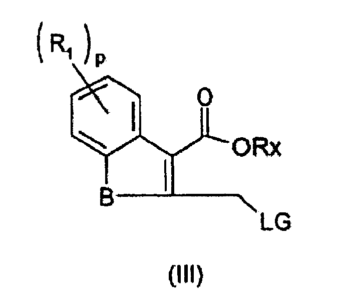 mit einer Verbindung der Formel (IV) oder einem Salz davon: 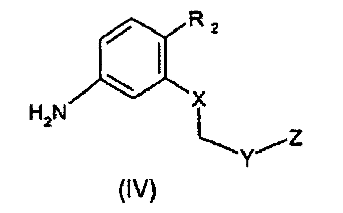 gefolgt von, falls angemessen, einer Behandlung des erhaltenen Zwischenprodukts mit AlMe₃ oder einem ähnlichen oxophilen Reagens;
und für entweder Verfahren (a) oder Verfahren (b) gegebenenfalls gefolgt von:
Entfernen etwaiger Schutzgruppen; und/oder
Umwandeln einer Verbindung der Formel (I) in eine andere Verbindung der Formel (I); und/oder
Bilden eines pharmazeutisch verträglichen Salzes.

11. Arzneimittel umfassend eine wie in einem der Ansprüche 1 bis 9 definierte Verbindung und einen pharmazeutisch verträglichen Träger oder Exzipienten.

12. Verfahren zur Herstellung eines Arzneimittels, wobei das Verfahren das Mischen einer wie in einem der Ansprüche 1 bis 9 beanspruchten Verbindung und eines pharmazeutisch verträglichen Trägers oder Exzipienten umfasst.

13. Verbindung nach einem der Ansprüche 1 bis 9 zur Verwendung als therapeutische Substanz.

14. Verbindung nach einem der Ansprüche 1 bis 9 zur Verwendung bei der Behandlung einer ZNS-Störung.

15. Verbindung nach Anspruch 14, wobei die Störung Depression oder Angst ist.

16. Verwendung einer Verbindung nach einem der Ansprüche 1 bis 9 bei der Herstellung eines Medikaments zur Verwendung bei der Behandlung einer ZNS-Störung.

17. Verwendung nach Anspruch 16, wobei die Störung Depression oder Angst ist.

## Revendications

1. Composé de formule (I) ou un de ses sels pharmaceutiquement acceptables : 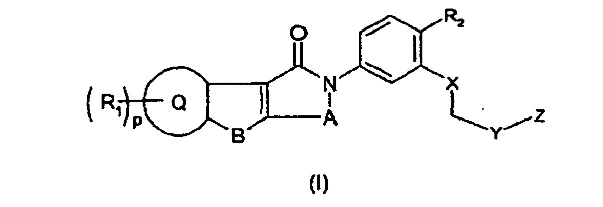 formule dans laquelle :
Q représente un groupe phényle ou un groupe hétéroaromatique hexagonal contenant au moins un atome d'azote ;
A représente un groupe - (CH₂-CH₂) -, -(CH=CH)-, -(CH₂)₃-, -C (CH₃)₂-, - (CH=CH-CH₂) -, ou un groupe - (CHR₃)-dans lequel R₃ représente un atome d'hydrogène, un atome d'halogène, un groupe hydroxy, cyano, nitro, alkyle en C₁ à C₆, cycloalkyle en C₃ à C₇, cycloalkyloxy en C₃ à C₇, halogénalkyle en C₁ à C₆, alkoxy en C₁ à C₆, halogénalkoxy en C₁ à C₆ ou alkylthio en C₁ à C₆ ;
B représente O, S ou un groupe NR₁₁, dans lequel R₁₁ représente un atome d'hydrogène ou un groupe alkyle en C₁ à C₆ ou alcanoyle en C₁ à C₆ qui peut être substitué avec un ou plusieurs substituants alkoxy en C₁ à C₆ ;
R₁ représente un atome d'halogène, un groupe cyano, alkyle en C₁ à C₆, cycloalkyle en C₃ à C₇, cycloalkyloxy en C₃ à C₇, alkoxy en C₁ à C₆, alkylthio en C₁ à C₆, hydroxy, amino, mono- ou di(alkyle en C₁ à C₆)amino, un groupe hétérocyclique tétra- à heptagonal lié par N, un groupe nitro, halogénalkyle en C₁ à C₆, halogénalkoxy en C₁ à C₆, aryle, aryl(alkyle en C₁ à C₆), aryl(alkyle en C₁ à C₆)oxy, aryl (alkyle en C₁ à C₆)thio ou COOR₄ ou COR₅ dans lequel R₄ et R₅ représentent indépendamment un atome d'hydrogène ou un groupe alkyle en C₁ à C₆, ou COR₆ dans lequel R₆ représente un groupe amino, mono- ou di(alkyle en C₁ à C₆)amino ou un groupe hétérocyclique tétra- à heptagonal lié par N ;
p est égal à 0, 1, 2 ou 3 ;
R₂ représente un atome d'hydrogène, un atome d'halogène, un groupe hydroxy, cyano, nitro, alkyle en C₁ à C₆, alcanoyle en C₁ à C₆, cycloalkyle en C₃ à C₇, cycloalkyloxy en C₃ à C₇, halogénalkyle en C₁ à C₆, alkoxy en C₁ à C₆, halogénalkoxy en C₁ à C₆, alkylthio en C₁ à C₆, amino, mono- ou di(alkyle en C₁ à C₆)amino ou un groupe hétérocyclique tétra- à heptagonal lié par N ;
X représente un atome d'oxygène ou de soufre, un groupe -CH₂- ou NR₈ dans lequel R₈ représente un atome d'hydrogène ou un groupe alkyle en C₁ à C₆ ;
Y représente une liaison simple, un groupe -CH₂-, -(CH₂)₂- ou -CH=CH- ; et
Z représente un groupe hétérocyclique lié par N ou un groupe hétérocyclique tétra- à heptagonal lié par C contenant au moins un atome d'azote, qui peut être substitué avec un ou plusieurs substituants alkyle en C₁ à C₆, amino, mono- ou di(alkyle en C₁ à C₆)amino, un groupe hétéroaromatique, un hétérocycle aromatique ou non aromatique lié par N ou C ou un groupe hétérocyclo(alkyle en C₁ à C₆) aromatique ou non aromatique qui peut être substitué avec un ou plusieurs substituants alkyle en C₁ à C₆ ;
ou bien Z représente un groupe -NR₉R₁₀ dans lequel R₉ et R₁₀ représentent indépendamment un atome d'hydrogène ou un groupe alkyle en C₁ à C₆.

2. Composé suivant la revendication 1, dans lequel A représente un groupe -CH₂-.

3. Composé suivant la revendication 1 ou 2, dans lequel p est égal à 1 ou 2 et le ou les groupes R₁ représentent des groupes halogéno, en particulier chloro ou fluoro.

4. Composé suivant la revendication 1, 2 ou 3, dans lequel R₂ représente un groupe alkoxy en C₁ à C₆ (en particulier méthoxy), halogéno ou cyano.

5. Composé suivant l'une quelconque des revendications 1 à 4, dans lequel X représente un atome d'oxygène.

6. Composé suivant l'une quelconque des revendications 1 à 5, dans lequel Y représente un groupe -CH₂-.

7. Composé suivant l'une quelconque des revendications 1 à 6, dans lequel Z représente un hétérocycle tétra- à heptagonal lié par N, facultativement substitué, en particulier un groupe pipéridyle.

8. Composé suivant la revendication 1, répondant à la formule (Ia) : 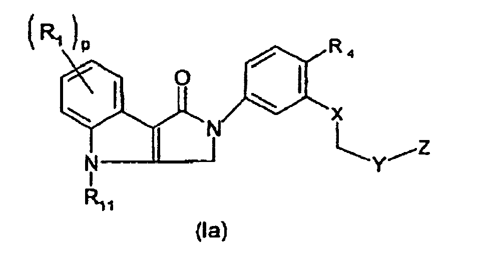 dans laquelle R₁, p, R₁₁, R₄, X, Y et Z répondent aux définitions figurant dans la revendication 1.

9. Composé suivant la revendication 1, qui est :
le chlorhydrate de 6-bromo-5-méthoxy-2-[4-méthoxy-3-(2-pipéridine-1-yl-éthoxy)phényl]-8-méthyl-1,8-dihydro-2H-2,8-diazacyclopenta[a]indène-3-one ;
le chlorhydrate de 5-méthoxy-2-[4-méthoxy-3-(2-pipéridine-1-yl-éthoxy)phényl]-8-méthyl-1,8-dihydro-2H-2,8-diazacyclopenta[a]indène-3-one ;
le chlorhydrate de 5,6-dichloro-2-[4-méthoxy-3-(2-pipéridine-1-yl-éthoxy)phényl]-8-méthyl-1,8-dihydro-2H-2,8-diazacyclopenta[a]indène-3-one ;
le chlorhydrate de 5-chloro-2-{4-méthoxy-3-[2-(4-méthyl-pipéridine-1-yl)-éthoxy]-phényl}-8-méthyl-1,8-dihydro-2H-2,8-diazacyclopenta[a]indène-3-one ;
le chlorhydrate de 2-[4-méthoxy-3-(2-pipéridine-1-yl-éthoxy)phényl]-1,2-dihydro-8-thia-2-aza-cyclopenta[a] indène-3-one ;
le chlorhydrate de 2-{4-méthoxy-3-[2-(4-méthyl-pipéridine-1-yl)-éthoxy]-phényl}-1,2-dihydro-8-thia-2-azacyclopenta[a]indène-3-one ;
le chlorhydrate de 2-[4-méthoxy-3-(2-pipéridine-1-yl-éthoxy)phényl]-8-méthyl-1,8-dihydro-2H-2,8-diazacyclopenta[a]indène-3-one ;
le chlorhydrate de 5-chloro-2-[4-méthoxy-3-(2-pipéridine-1-yl-éthoxy)phényl]-8-méthyl-1,8-dihydro-2H-2,8-diazacyclopenta[a]indène-3-one ;
le chlorhydrate de 6-chloro-2-[4-méthoxy-3-(2-pipéridine-1-yl-éthoxy)phényl]-8-méthyl-1,8-dihydro-2H-2,8-diazacyclopenta[a]indène-3-one ;
le chlorhydrate de 6-fluoro-2-[4-méthoxy-3-(2-pipéridine-1-yl-éthoxy)phényl]-8-méthyl-1,8-dihydro-2H-2,8-diazacyclopenta[a]indène-3-one ;
le chlorhydrate de 2-{4-méthoxy-3-[2-(4-méthyl-pipéridine-1-yl)-éthoxy]-phényl}-1,8-diméthyl-1,8-dihydro-2H-2,8-diazacyclopenta[a]indène-3-one ;
le chlorhydrate de 2-{4-méthoxy-3-[2-(4-méthyl-pipéridine-1-yl)-éthoxy]-phényl}-8-méthyl-1,8-dihydro-2H-2,8-diazacyclopenta[a]indène-3-one ;
le chlorhydrate de 6-chloro-2-{4-méthoxy-3-[2-(4-méthyl-pipéridine-1-yl) -éthoxy] -phényl}-8-méthyl-1,8-dihydro-2H-2,8-diazacyclopenta[a]indène-3-one ;
le chlorhydrate de 5-fluoro-2-{4-méthoxy-3-[2-(4-méthyl-pipéridine-1-yl)-éthoxy]-phényl}-8-méthyl-1,8-dihydro-2H-2,8-diazacyclopenta[a]indène-3-one ;
le chlorhydrate de 6-fluoro-2-{4-méthoxy-3-[2-(4-méthyl-pipéridine-1-yl)-éthoxy]-phényl}-8-méthyl-1,8-dihydro-2H-2,8-diazacyclopenta[a]indène-3-one ;
le chlorhydrate de 7-fluoro-2-{4-méthoxy-3-[2-(4-méthyl-pipéridine-1-yl)-éthoxy]-phényl}-8-méthyl-1,8-dihydro-2H-2,8-diazacyclopenta[a]indène-3-one ;
ou un de ses sels pharmaceutiquement acceptables.

10. Procédé pour la préparation d'un composé suivant la revendication 1, procédé qui comprend :
(a) la réaction d'un composé de formule (II) : 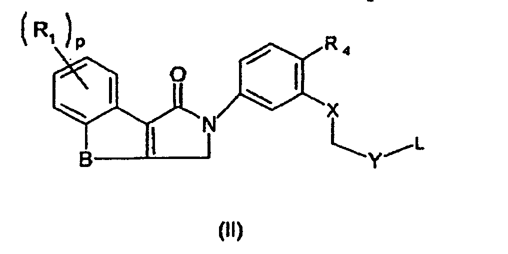 dans laquelle R₁, R₂, p, A, X et Y répondent aux définitions pour la formule (I) et L représente un groupe partant, avec un composé de formule (III) :
Z-H (III)
dans laquelle Z répond à la définition mentionnée pour la formule (I) ; ou
(b) la réaction d'un composé de formule (III) : 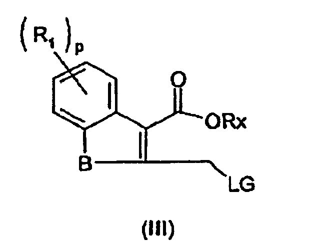 avec un composé de formule (IV) ou un de ses sels : 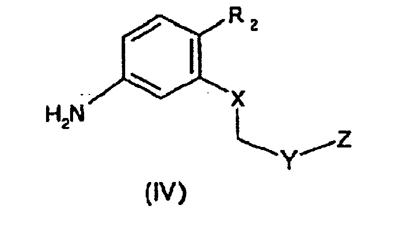 avec ensuite, de manière appropriée, un traitement de l'intermédiaire obtenu avec AlMe₃ ou un réactif oxophile similaire ;
et, pour le procédé (a) ou le procédé (b), ensuite facultativement :
l'élimination de n'importe quels groupes protecteurs ; et/ou
la conversion d'un composé de formule (I) en un autre composé de formule (I) ; et/ou
la formation d'un sel pharmaceutiquement acceptable.

11. Composition pharmaceutique comprenant un composé tel que défini dans l'une quelconque des revendications 1 à 9, et un support ou excipient pharmaceutiquement acceptable.

12. Procédé pour la préparation d'une composition pharmaceutique, procédé comprenant le mélange d'un composé suivant l'une quelconque des revendications 1 à 9, et d'un support ou excipient pharmaceutiquement acceptable.

13. Composé suivant l'une quelconque des revendications 1 à 9, destiné à être utilisé comme substance thérapeutique.

14. Composé suivant l'une quelconque des revendications 1 à 9, destiné à être utilisé dans le traitement d'un trouble du SNC.

15. Composé suivant la revendication 14, dans lequel le trouble est la dépression ou l'anxiété.

16. Utilisation d'un composé suivant l'une quelconque des revendications 1 à 9, dans la production d'un médicament destiné à être utilisé dans le traitement d'un trouble du SNC.

17. Utilisation suivant la revendication 16, dans laquelle le trouble est la dépression ou l'anxiété.
